# EUROPEAN PATENT SPECIFICATION

(11) **EP 3 165 235 B1**
(45) Date of publication and mention of the grant of the patent: **30.10.2019**
(21) Application number: 15800403.6
(22) Date of filing: 29.05.2015
(51) Int. Cl.: A61K 39/00, A61K 38/00, A61P 33/14

(54) **PROTEINS FOR USE IN CONTROLLING RED MITES**
PROTEINE ZUR VERWENDUNG IN DER BEKÄMPFUNG VON ROTEN MILBEN
PROTÉINE POUR L'UTILISATION DANS LA LUTTE CONTRE DERMANYSSUS GALLINAE

(30) Priority: 30.05.2014 JP 2014113479
(43) Date of publication of application: 10.05.2017
(73) Proprietor: National University Corporation Hokkaido University, Sapporo-shi, Hokkaido 060-0808 (JP)
(72) Inventor: OHASHI Kazuhiko, Sapporo-shi Hokkaido 060-0808 (JP); MURATA Shiro, Sapporo-shi Hokkaido 060-0808 (JP); ISEZAKI Masayoshi, Sapporo-shi Hokkaido 060-0808 (JP); KONNAI Satoru, Sapporo-shi Hokkaido 060-0808 (JP); TANIGUCHI Ayaka, Sapporo-shi Hokkaido 060-0808 (JP); HOJOH Takumi, Sapporo-shi Hokkaido 060-0808 (JP)
(74) Representative: Prüfer & Partner mbB Patentanwälte · Rechtsanwälte
(86) International application number: PCT/JP2015/065600
(87) International publication number: WO 2015/182754

(56) References cited:
- WO-A2-2013/014408
- CN-A- 102 051 363
- US-A- 6 103 514
- MASAMI ISEZAKI ET AL.: 'Wakumo Dermanyssus gallinae Yurai Hatsugen Idenshi no Morateki Kaiseki' JAPANESE SOCIETY OF VETERIANY SCIENCE GAKUJUTSU SHUKAI KOEN YOSHISHU vol. 154, 2012, page 203, XP008185568
- KATHRYN BARTLEY ET AL.: 'Assessment of cathepsin D and L-like proteinases of poultry red mite, Dermanyssus gallinae (De Geer), as potential vaccine antigens' PARASITOLOGY vol. 139, no. 6, 2012, pages 755 - 65, XP009167278
- ONDREJ HAJDUSEK ET AL.: 'Knockdown of proteins involved in iron metabolism limits tick reproduction and development' PROC NATL ACAD SCI U S A vol. 106, no. 4, 2009, pages 1033 - 8, XP002562990

## Description

### Technical Field

The present invention relates to proteins for use in a method for controlling red mites (Dermanyssus gallinae), which are ectoparasites that parasitize domestic animals, particularly chickens, and to a novel protein that can be used therein.

### Background Art

Damage resulting from bloodsucking by red mites is a major problem in poultry farms (particularly of hens for eggs) in the world. Red mites cause to decrease the egg productivity of hens, for example, due to anemia, a decrease in the egg-laying rate and a decrease in the immune response of hens resulting from their bloodsucking. Red mites are also reported to be involved in the transmission of various pathogens.

Current methods for controlling red mites include, for example, thorough cleaning of poultry houses, and spraying of insecticides. However, since red mites stay in gaps like cracks in a poultry house while they do not suck blood, it is difficult to completely control the red mites staying in such gaps by cleaning or by chemical spraying. Furthermore, chemical spraying may cause the chemical drug to remain on the product, and in addition to that, is associated with a problem of generating resistant red mites to the chemical drug, as recently reported. Since the current methods for controlling redmites, particularly those by chemical spraying have such problems, there is a need for a new method for controlling red mites, other than chemical spraying.

A vaccine against red mites is a promising means as an alternative method to chemical spraying for controlling redmites. Developmental research on anti-tick vaccines for ixodid ticks, which are bloodsucking ectoparasites of domestic animals as well as red mites, has already precedingly been made.

Anti-tick vaccines include, for example, those in which a molecule that is injected from a tick into a host is used as a vaccine antigen, and those in which a molecule that is expressed in the intestinal tract of a tick and involved in maintaining the homeostasis in the tick is used as a vaccine antigen. In the former case, the function of the factor that has been injected from the tick into the host is inhibited by an immune response within the host, thereby the tick is prevented from bloodsucking and is controlled. In the latter case, an immunoactive substance that has been introduced from the host into the tick with bloodsucking inhibits the function of the target molecule within the body of the tick, thereby the tick is controlled (Non-Patent Literatures 1 and 2). An anti-tick vaccine belonging to the former type is TickGARD (registered trademark) PLUS, which is commercially available from Hoechst.

In recent years, there have also been made research and development on vaccines against red mites. For example, a vaccine test was performed in which insoluble and soluble fractions from adult red mites are used as antigens (Non-Patent Literature 3). It has also been reported that a vaccine test showed a certain effect in which a recombinant protein encoded by the gene identified to be expressed in red mites is used as an antigen (Non-Patent Literatures 4 and 5). Furthermore, there have also been reported molecules that can be candidate antigens for vaccines, such as antioxidative enzymes anddrugmetabolizing enzymes in red mites (Non-Patent Literatures 6 and 7).

Since ixodid ticks parasitize a host and continue to suck the host' s blood for several days, vaccines which prevent ticks from bloodsucking by an immune response within the host are also effective. In contrast, the period during bloodsucking of red mites is from five minutes to, at longest, about one hour, and thus red mites stay on chicken individuals only for extremely shorter periods, in comparison to ixodid ticks. For this reason, as vaccines against red mites that can be expected to be highly effective in controlling red mites, those that control red mites by an immunoactive substance introduced from a host into the body of the red mite with bloodsucking would be more advantageous than those that control red mites by inhibiting the function of a molecule derived from red mites through an immune response within the host. However, currently, no effective vaccine antigen has been found which can control red mites through such a mechanism.

### Citation List

### Non-Patent Literatures

Non-Patent Literature 1: Onuma et al., Journal of Veterinary Medicine, 2004, Vol. 57, 753-757.
Non-Patent Literature 2: Willadsen et al., Parasitology, 2004, Vol. 129, S367-S387.
Non-Patent Literature 3 : Wright et al. , Exp. Appl. Acarol., 2009, Vol. 48, 81-91.
Non-Patent Literature 4: Bartley et al., International Journal for Parasitology, 2009, Vol. 39, 447-456.
Non-Patent Literature 5: Bartley et al., Parasitology, 2012, Vol. 139, 755-765.
Non-Patent Literature 6: Isezaki et al., September 18, 2010, the 150th Japanese Society of Veterinary Science, presentation number C-52.
Non-Patent Literature 7: Isezaki et al., September 15, 2012, the 154th Japanese Society of Veterinary Science, presentation number C-2.

### Summary of Invention

### Technical Problem

The present invention is defined by the claims. The present invention aims at providing controlling red mites by eliciting an immune response against them in a domestic animal to inhibit the function of the target molecule within the body of the red mites, and a vaccine antigen and a vaccine against red mites that can be used therefor.

### Solution to Problem

The present inventors have revealed the expression of genes encoding novel proteins characteristic for red mites by gene expression analysis, and have accomplished the invention, of which embodiments are set forth below. The present disclosure comprising the invention relates to the claimed proteins for the use in the following methods:
(1) A method for eliciting an immune response specific for red mites in a domestic animal, comprising the step of administering to the domestic animal a protein consisting of the amino acid sequence set forth in SEQ ID NO. 1, a protein consisting of the amino acid sequence set forth in SEQ ID NO. 2, and/or an immunological equivalent thereof.
(2) The method according to (1), wherein the immunological equivalent is a polypeptide fragment of a protein consisting of the amino acid sequence set forth in SEQ ID NO. 1 or a protein consisting of the amino acid sequence set forth in SEQ ID NO. 2.
(3) The method according to (1), wherein the immunological equivalent is
   a protein consisting of an amino acid sequence in which one to several amino acids in the amino acid sequence set forth in SEQ ID NO. 1 are deleted or substituted,
   a protein consisting of an amino acid sequence in which one to several amino acids are inserted in the amino acid sequence set forth in SEQ ID NO. 1,
   a protein consisting of an amino acid sequence in which one to several amino acids in the amino acid sequence set forth in SEQ ID NO. 2 are deleted or substituted,
   a protein consisting of an amino acid sequence in which one to several amino acids are inserted in the amino acid sequence set forth in SEQ ID NO. 2, or
   a polypeptide fragment thereof.
(4) The method according to (1), wherein the immunological equivalent is a fusion protein consisting of an amino acid sequence in which an optional amino acid(s) is/are added at the N- and/or C-terminus of the amino acid sequence of any of the following:
   the amino acid sequence set forth in SEQ ID NO. 1,
   an amino acid sequence in which one to several amino acids in the amino acid sequence set forth in SEQ ID NO. 1 are deleted or substituted,
   an amino acid sequence in which one to several amino acids are inserted in the amino acid sequence set forth in SEQ ID NO. 1,
   the amino acid sequence set forth in SEQ ID NO. 2,
   an amino acid sequence in which one to several amino acids in the amino acid sequence set forth in SEQ ID NO. 2 are deleted or substituted,
   an amino acid sequence in which one to several amino acids are inserted in the amino acid sequence set forth in SEQ ID NO. 2,
   or a partial amino acid sequence thereof.
(5) A protein consisting of the amino acid sequence of any of a) to d) described below, or a polypeptide fragment consisting of a partial amino acid sequence of the amino acid sequence of a) to c) described below, wherein the protein or the polypeptide fragment is an immunological equivalent of a protein consisting of the amino acid sequence set forth in SEQ ID NO. 1:
   a) the amino acid sequence set forth in SEQ ID NO. 1,
   b) an amino acid sequence in which one to several amino acids in the amino acid sequence set forth in SEQ ID NO. 1 are deleted or substituted,
   c) an amino acid sequence in which one to several amino acids are inserted in the amino acid sequence set forth in SEQ ID NO. 1,
   d) an amino acid sequence in which an optional amino acid (s) is/are added at the N- and/or C-terminus of the amino acid sequence or a partial amino acid sequence thereof of any of a) to c) .
(6) A protein consisting of the amino acid sequence of any of e) to h) described below, or a polypeptide fragment consisting of a partial amino acid sequence of the amino acid sequence of e) to g) described below, wherein the protein or the polypeptide fragment is an immunological equivalent of a protein consisting of the amino acid sequence set forth in SEQ ID NO. 2:
   e) the amino acid sequence set forth in SEQ ID NO. 2,
   f) an amino acid sequence in which one to several amino acids in the amino acid sequence set forth in SEQ ID NO. 2 are deleted or substituted,
   g) an amino acid sequence in which one to several amino acids are inserted in the amino acid sequence set forth in SEQ ID NO. 2,
   h) an amino acid sequence in which an optional amino acid (s) is/are added at the N- and/or C-terminus of the amino acid sequence or a partial amino acid sequence thereof of any of e) to g) .
(7) A nucleic acid encoding the protein or the polypeptide fragment thereof as defined in (5) or (6).
(8) An expression vector comprising the nucleic acid according to (7).
(9) A vaccine against red mites for use in a domestic animal, comprising the protein or the polypeptide fragment as defined in (5) and/or (6), or the nucleic acid as defined in (7) and/or the expression vector as defined in (8).

### Advantageous Effects of Invention

According to the present invention, an immune response specific for red mites can be elicited in a domestic animal, and red mites can be controlled by inhibiting the function of the target molecule within the red mites that has sucked the blood of the domestic animal.

### Brief Description of Drawings

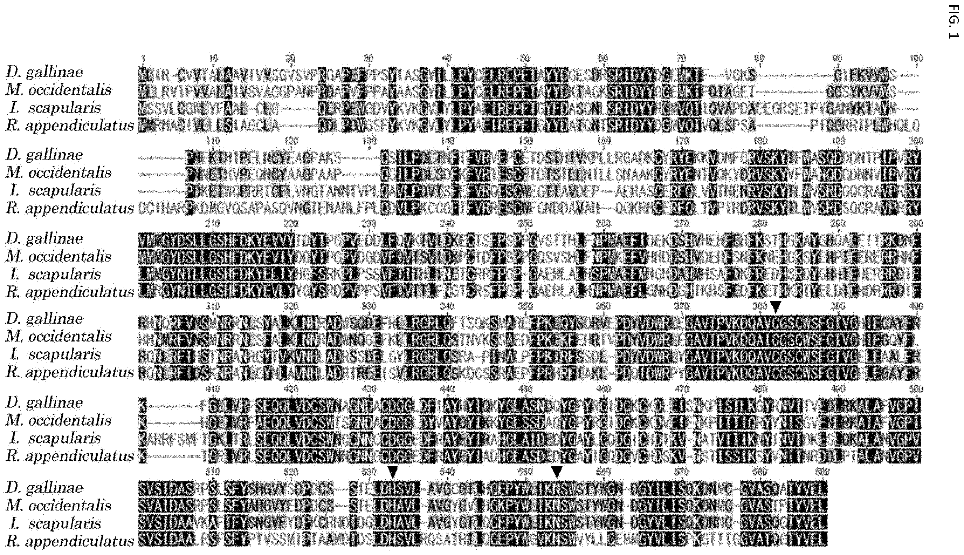

Fig. 1 represents an alignment of the amino acid sequences of red mite (Dermanyssus gallinae) cathepsin L-2 set forth in SEQ ID NO. 1 and cathepsin L from several species of ticks and mites. In the figure, the inverted closed triangles represent a motif sequence characteristic for cathepsin L.

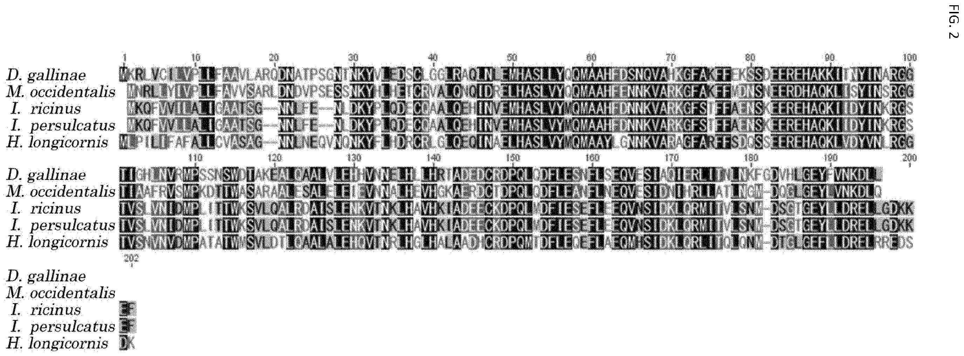

Fig. 2 represents an alignment of the amino acid sequences of red mite (Dermanyssus gallinae) ferritin 2 set forth in SEQ ID NO. 2 and ferritin 2 from several species of ticks and mites.

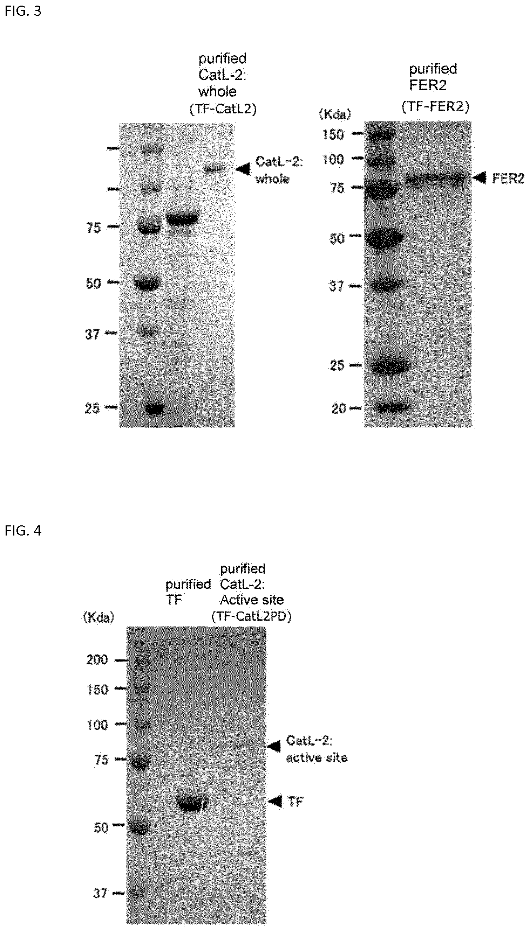

Fig. 3 represents a photograph of a gel after SDS-PAGE of a fusion protein of Dermanyssus gallinae cathepsin L-2 and Escherichia coli-derived Trigger Factor on the left panel, and a photograph of a gel after SDS-PAGE of a fusion protein of Dermanyssus gallinae ferritin 2 and Escherichia coli-derived Trigger Factor on the right panel.
Fig. 4 represents a photograph of a gel after SDS-PAGE of a fusion protein of a portion containing the active site of Dermanyssus gallinae cathepsin L-2 and an Escherichia coli-derived Trigger Factor.

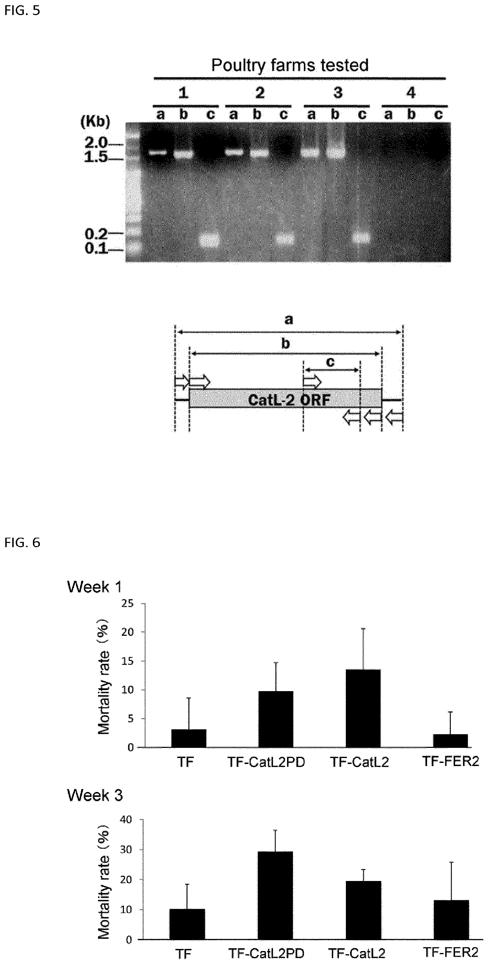

Fig. 5 represents a photograph of agarose gel electrophoresis showing the results of RT-PCR performed by using total RNAs from three samples of Dermanyssus gallinae mites collected in different poultry farms and a control sample from Ornithonyssus sylviarum mites as a template, and primer sets a) toc) designed to amplify the nucleic acid encoding Dermanyssus gallinae cathepsin L-2. In the upper panel, lanes 1 to 3 correspond to D. gallinae samples, and lane 4 corresponds to an O. sylviarum sample. The lower panel is a schematic representation showing the relation between the locations of the primers of each primer sets and the fragments to be amplified with the primer sets.
Fig. 6 represents graphs showing the mortality rate (the number of Dermanyssus gallinae mites killed by bloodsucking relative to the number of Dermanyssus gallinae mites having sucked blood) of Dermanyssus gallinae mites having sucked a test blood prepared from chickens immunized with Dermanyssus gallinae cathepsin L-2 or ferritin 2 at weeks 1 and 3 after bloodsucking.

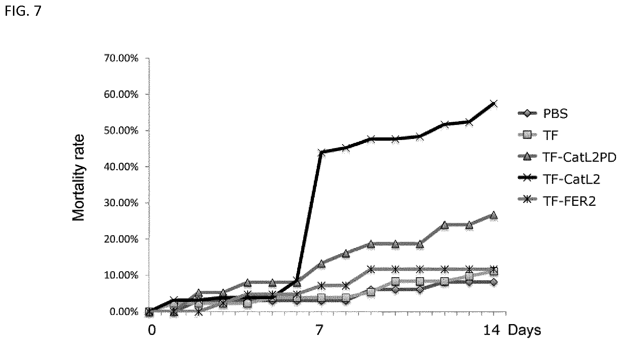

Fig. 7 represents graphs showing the mortality rate (the number of Dermanyssus gallinae mites killed by bloodsucking relative to the total number of both Dermanyssus gallinae mites having and not having sucked blood) of Dermanyssus gallinae mites having sucked a test blood prepared from chickens immunized with Dermanyssus gallinae cathepsin L-2 or ferritin 2 for a period up to 14 days after bloodsucking.

### Description of Embodiments

The present disclosure comprising the invention is directed to a method for eliciting an immune response specific for red mites (Dermanyssus gallinae) in a domestic animal, comprising the step of administering to the domestic animal a protein consisting of the amino acid sequence set forth in SEQ ID NO. 1, a protein consisting of the amino acid sequence set forth in SEQ ID NO. 2, and/or an immunological equivalent thereof as defined by the claims.

A protein consisting of the amino acid sequence set forth in SEQ ID NO. 1 was ascertained to be cathepsin L, a kind of a cysteine protease, on the basis of a plurality of characteristic motif sequences contained in its amino acid sequence. In relation to this, the nucleic acid cloned in the present invention (SEQ ID NO. 3) encodes a protein having a signal sequence of 18 amino acid residues added at the N terminus of the amino acid sequence of SEQ ID NO. 1.

From red mites, a gene encoding cathepsin L had already been cloned (Bartley et al., Non-Patent Literature 5, supra), and has been registered in GenBank (http://www.ncbi.nlm.nih.gov/genbank/) under Accession No. CCC33064.1. However, the amino acid sequence encoded by the base sequence of SEQ ID NO. 3 shows only an identity of 30.6% with that of the Accession No. CCC33064.1. The identity of an amino acid sequence in the specification means the highest percent sequence identity obtained by an alignment of sequences to be compared using an appropriate software program usually used by those skilled in the art, such as NCBI BLAST search (http://blast.ncbi.nlm.nih.gov/) .

A homology search in GenBank was performed using the amino acid sequence encoded by the base sequence of SEQ ID NO. 3 as a query sequence, and it was ascertained that Metaseiulus occidentalis cathepsin L (Accession No. XP_003745143.1, SEQ ID NO. 5) has an identity of 72.9%, Ixodes scapularis cathepsin L (Accession No. XP_002403652.1, SEQ ID NO. 6) has an identity of 51.8%, and Rhipicephalus appendiculatus cathepsin L (AccessionNo. AAO60045.1, SEQ ID NO. 7) has an identity of 46. 6%, respectively with the query sequence. However, no known amino acid sequences showing an identity higher than those of these sequences were detected in the members of the order Acari.

Based on these results, a protein consisting of the amino acid sequence set forth in SEQ ID NO. 1 was ascertained to be a novel cathepsin L that is specifically expressed in the red mite Dermanyssus gallinae. Hereinafter, the cathepsin in the present invention consisting of the amino acid sequence set forth in SEQ ID NO. 1 is referred to as cathepsin L-2 and abbreviated as DgCatL-2, and the known Dermanyssus gallinae cathepsin L registered under Accession No. CCC33064.1 is referred to as cathepsin L-1 and abbreviated as DgCatL-1.

A protein consisting of the amino acid sequence set forth in SEQ ID NO. 2 was ascertained to be ferritin 2, which is involved in the transportation and storage of iron, on the basis of characteristic motif sequences contained in its amino acid sequence. Ferritin is an iron-storage protein that is widely present in species ranging from mites and ticks to mammalian animals : it has been reported that mites and ticks have ferritins 1 and 2, whereas mammalian animals have a ferritin corresponding to ferritin 1 of mites and ticks. The ferritin in the present invention consisting of the amino acid sequence set forth in SEQ ID NO. 2 is a protein corresponding to ferritin 2. In relation to this, the nucleic acid that was cloned in the present invention (SEQ ID NO. 4) encodes a protein having a signal sequence of 18 amino acid residues added at the N terminus of the amino acid sequence of SEQ ID NO. 2.

A homology search in GenBank was performed using the amino acid sequence encoded by the base sequence of SEQ ID NO. 4 as a query sequence, and it was ascertained that Metaseiulus occidentalis ferritin 2 (Accession No. XP_003737398.1, SEQ ID NO. 8) has an identity of 59.0%, Ixodes ricinus ferritin 2 (Accession No. ACJ70653 .1, SEQ ID NO. 9) has an identity of 49.5%, Ixodes persulcatus ferritin 2 (Accession No. AGX01000.1, SEQ ID NO. 10) has an identity of 49. 5%, and Haemaphysalis longicornis ferritin 2 (Accession No. BAN13552.1, SEQ ID NO. 11) has an identity of 48.2%, respectively with the query sequence. However, no known amino acid sequences showing an identity higher than those of these sequences were detected in the members of the order Acari.

Based on these results, a protein consisting of the amino acid sequence set forth in SEQ ID NO. 2 was ascertained to be a novel ferritin 2 that is specifically expressed in the red mite Dermanyssus gallinae. Hereinafter, the ferritin 2 in the present invention consisting of the amino acid sequence set forth in SEQ ID NO. 2 is abbreviated as DgFER2.

The above-described DgCatL-2 and DgFER2 are each assumed to be a protein involved in the digestion and absorption of the host's blood that has been incorporated into the body of a red mite. It is expected that inhibition of the function of these proteins makes the nutrient intake of red mites insufficient, thereby the red mites can be controlled. Specifically, it is expected that inducing the production within the host of various materials exhibiting an immunological response specific for DgCatL-2 or DgFER2, such as specific antibodies, killer T cells, helper T cells and NKT cells, that is to say, elicitation of Dermanyssus gallinae-specific immune response in the host, allows red mites to be controlled.

In addition to DgCatL-2 and DgFER2, immunological equivalents thereof can also be used in the present invention. An immunological equivalent as used in the present invention means an immunogenic protein or polypeptide that is capable of eliciting Dermanyssus gallinae-specific immune response directed to DgCatL-2 or DgFER2 as a target antigen in a domestic animal. Preferable, non-limiting examples of such an immunological equivalent can include the following b) to d), and f) to h).
b) A protein consisting of an amino acid sequence in which one to several amino acids in the amino acid sequence set forth inSEQ IDNO. 1 are deleted or substituted (a protein corresponding to a deletion or substitution variant of DgCatL-2) or a polypeptide fragment thereof.
c) A protein consisting of an amino acid sequence in which one to several amino acids are inserted in the amino acid sequence set forth in SEQ ID NO. 1 (a protein corresponding to an insertion variant of DgCatL-2) or a polypeptide fragment thereof.
d) A protein consisting of an amino acid sequence in which an optional amino acid (s) is/are added at the N- and/or C-terminus of the amino acid sequence of any of DgCatL-2 or the proteins described in the above b) or c) or polypeptide fragments thereof (a fusion protein of DgCatL-2 or the protein described in the above b) or c) with an another protein or polypeptide or a tag sequence).
f) A protein consisting of an amino acid sequence in which one to several amino acids in the amino acid sequence set forth in SEQ ID NO. 2 are deleted or substituted (a protein corresponding to a deletion or substitution variant of DgFER2) or a polypeptide fragment thereof.
g) A protein consisting of an amino acid sequence in which one to several amino acids are inserted in the amino acid sequence set forth in SEQ ID NO. 2 (a protein corresponding to an insertion variant of DgFER2) or a polypeptide fragment thereof.
h) A protein consisting of an amino acid sequence in which an optional amino acid (s) is/are added at the N- and/or C-terminus of the amino acid sequence of any of DgFER2 or the proteins described in the above f) or g) or polypeptide fragments thereof (a fusion protein of DgFER2 or the protein described in the above f) or g) with an another protein or polypeptide or a tag sequence) .

Representative examples of an optional amino acid sequence to be added as a portion of the above-mentioned fusion proteins in the present invention can include, but are not limited to, tag sequences such as DYKDDDDK and His tags, and amino acid sequences of proteins, such as Escherichia coli trigger factor, keyhole limpet hemocyanin, hemagglutinin, c-myc, fluorescent proteins, and glutathione S-transferase.

In the present disclosure comprising the invention, DgCatL-2, DgFER2, and/or immunological equivalents thereof, preferably proteins described in the above b) to d), and f) to h), are used as an antigen for eliciting Dermanyssus gallinae-specific immune response in a host. An immunological equivalent is not necessarily required to retain the protease activity of DgCatL-2 or the function of DgFER2 as an iron-storage protein in the present invention.

Therefore, for example, in immunological equivalents corresponding to DgCatL-2 or DgFER2 variants, there are no particular limitations on the site of amino acid substitution, deletion, and/or insertion, the type of amino acid residues after mutation, and amino acid sequences to be added except the percentage of the sequence identity. The amino acid sequence of an immunological equivalent variant has an identity of at least 80% or higher, or 90% or higher, furthermore desirably 95% or higher, with the amino acid sequence set forth in SEQ ID NO. 1 or 2. Polypeptide fragments have no less than 30 amino acid residues.

In addition, DgCatL-2, DgFER2 and immunological equivalents thereof may be modified with fluorescent substances such as FITC, crosslinking agents, and other known chemical substances, as long as they retain immunogenicity. The selection of chemical substances with which proteins can be modified, and modifications therewith can be within normal capability of a person skilled in the art.

DgCatL-2, DgFER2 and immunological equivalents thereof in the present invention can be produced using genetic recombination procedures known or well-known to those skilled in the art. Such genetic recombinationprocedures canbe carried out using procedures described in a variety of textbooks and handbooks, typically in Sambrook et al., Molecular Cloning: A Laboratory Manual, 3rd ed., Cold Spring Harbor Press (2001), and AuSubel et al., Current Protocols in Molecular Biology, Greene Publishing Associates (1992), and commonly used by those skilled in the art. Commercially available experimental kits or reagents can be used according to the protocols made by their manufacturers.

DgCatL-2, DgFER2 and immunological equivalents thereof in the present invention can be produced as recombinant proteins by incorporating a nucleic acid which contains the base sequence that encodes each of these into an appropriate expression vector, and expressing the nucleic acid in a host cell, by means of genetic engineering methods commonly used. The expression vector may further contain any functional base sequence for regulating transcription and expression, such as a promoter sequence, an operator sequence, a ribosome binding site, a polyadenylation signal, and an enhancer. These functional base sequences can be operably linked to the above-described nucleic acid.

As described above, in the present invention, DgCatL-2, DgFER2 and immunological equivalents thereof are not necessarily required to retain physiological activities, such as protease activity or a function as an iron-storage protein. Therefore, when these proteins are produced by genetic engineering procedures, they are not required to be expressed as proteins retaining the above-mentioned physiological activity. For example, DgCatL-2, DgFER2 and immunological equivalents thereof may be produced in the form of so-called inclusion bodies that may be generated when a heterologous protein is expressed using Escherichia coli as a host cell.

Relatively small proteins like polypeptide fragments can be synthesized by means of chemical synthesis methods well-known to those skilled in the art, such as Fmoc (fluorenylmethyloxycarbonyl) and tBoc (t-butyloxycarbonyl) methods, or using commercially available peptide synthesizers.

Furthermore, DgCatL-2, DgFER2 and immunological equivalents thereof can be recombinantly expressed with a signal sequence added at the N terminus of each of these. Examples of a signal sequence include the signal sequences of DgCatL-2 itself (SEQ ID NO. 24) and DgFER2 itself (SEQ ID NO. 25), which are preferably used, whereas a different signal sequence can be used as appropriate, depending on the host cell to be used.

A method of the present disclosure comprising the invention for eliciting an immune response specific for red mites in a domestic animal comprises the step of administering to the domestic animal a protein consisting of the amino acid sequence set forth in SEQ ID NO. 1, a protein consisting of the amino acid sequence set forth in SEQ ID NO. 2, and/or an immunological equivalent thereof, that is, DgCatL-2, DgFER2 and/or an immunological equivalent thereof as described above.

The present invention can be widely applied to domestic animals in which controlling of red mites is required, and is preferably applied to poultry, such as chickens, jungle fowls, domestic ducks, ducks, geese, wild geese, turkeys, guinea fowls, pheasants, golden pheasants, and quails, particularly chickens, including ones for eggs, for food, and for ornament.

When administered to a domestic animal, DgCatL-2, DgFER2, and/or an immunological equivalent thereof is preferably formulated into and used as appropriate dosage forms in combination with excipients, adjuvants, and other pharmaceutically acceptable ingredients. Such formulation can be within normal capability of a person skilled in the art. For administration routes, suitable methods can be selected depending on dosage forms, and such methods can include eye drop administration, nasal drip administration, spray administration, atomization administration, administration in drinking water, oral administration, mixed-feed administration, administration into eggs, puncture, intramuscular injection, subcutaneous injection, and others. Particularly preferable methods for administration in the present invent ion are puncture, intramuscular injection, subcutaneous injection, and others.

Elicitation of Dermanyssus gallinae-specific immune response in a domestic animal to which DgCatL-2, DgFER2 and/or an immunological equivalent thereof has been administered can be confirmed by detecting the production of, for example, a specific antibody, killer T cells, helper T cells, and NKT cells that exhibit an immune response to DgCatL-2 or DgFER2, within the host or in samples of the peripheral blood or lymph fluid collected from the host. Detection of such an antibody or types of cells can be within normal capability of a person skilled in the art.

The present invention also provides DgCatL-2, DgFER2, and an immunological equivalent thereof, and nucleic acids, expression vectors, and transformed host cells encoding the same that can be used in the method for eliciting an immune response specific for red mites in a domestic animal. These are useful as vaccines that allow for elicitation of Dermanyssus gallinae-specific immune response in a host. Details of DgCatL-2, DgFER2, and an immunological equivalent thereof, and the nucleic acids, expression vectors, and host cells, etc., and in addition, methods producing them, and others are as described above, including their preferable examples. An above-mentioned nucleic acid or expression vector, particularly an expression vector that functions within the body of poultry such as chickens can also be used as a so-called DNA vaccine against red mites.

The ability to control red mites through Dermanyssus gallinae-specific immune response that is elicited by administering to a domestic animal DgCatL-2, DgFER2 and an immunological equivalent thereof as described in the present invention can be evaluated by means of simple methods. Specifically, it can be evaluated, for example, by a method which comprises preparing a blood collected from chickens reared for a period of time usually required to induce immune response after administration of DgCatL-2, DgFER2 and an immunological equivalent thereof, allowing red mites to suck the collected blood, and then determining deaths of the mites over time. In this method, since chicken skin is required for bloodsucking of red mites, it is preferable to use an artificial bloodsucking system in which a piece of chicken skin is provided as a portion of a dividing wall, the blood is retained to be in contact with the dividing wall and red mites are placed on the opposite side of the dividing wall, whereby the red mites are allowed to be kept with free bloodsucking through the dividing wall. Such a system can be constructed within normal capability of a person skilled in the art, and modified and used as appropriate.

The present invention will be further described in detail with reference to Examples which follow.

### Examples

### <Example 1> Cloning of DgCatL-2

### 1) cDNA synthesis

Routine procedures were used to synthesize cDNAs from the mRNA fraction extracted from mite bodies of Dermanyssus gallinae . Using cDNAs as a template, 3' RACE was performed to amplify a gene encoding Dermanyssus gallinae acetylcholinesterase (Ace) with the 3' RACE kit (3' RACE System for Rapid Amplification of cDNA Ends) from Life Technologies. The 1st round of PCR was performed using a primer F1 (SEQ ID NO. 12) designed to amplify a gene encoding the Ace and a primer AUAP (a primer contained in the 5' RACE and 3' RACE kit (3' RACE / 5' RACE System for Rapid Amplification of cDNA Ends) from Life Technologies). Furthermore, nested-PCR was performed using the amplified DNA fragments as a template, and another primer F2 (SEQ ID NO. 13) designed to amplify a gene encoding the Ace and the primer AUAP.

### 2) Sequencing of DNA fragment and homology search

A 1.2-kbp amplification fragment obtained by two rounds of PCR was sequenced, and the amino acid sequence encoded by this fragment was searched with BLAST to analyze the conservation of characteristic motifs or domains . The analysis revealed that in the encoded amino acid sequence, there were three motifs of amino acid sequences characteristic in the cathepsin L family (which correspond to the portions indicated by the inverted closed triangles in Fig. 1).

### 3) Determination of the full-length sequence

Using the obtained base sequence, the 5' RACE kit (5' RACE System for Rapid Amplification of cDNA Ends) from Life Technologies was used to carry out 5' RACE to identify a full-length cDNA. Sequencing of the cDNA with a DNA sequencer determined a base sequence set forth in SEQ ID NO. 3, which encodes a protein having a signal sequence of 18 amino acids added at the N terminus of the amino acid sequence set forth in SEQ ID NO. 1.

Furthermore, a BLAST homology search in GenBank was performed using the amino acid sequence (including the signal sequence) encoded by the determined base sequence as a query sequence. The results of the BLAST homology search revealed that Metaseiulus occidentalis cathepsin L (Accession No. XP_003745143.1) has an identity of 72.9%, Ixodes scapularis cathepsin L (Accession No. XP_002403652.1) has an identity of 51.8%, and Rhipicephalus appendiculatus cathepsin L (Accession No. AAO60045.1) has an identity of 46.6%, respectively with the query sequence. The result of an alignment of these four amino acid sequences is shown in Fig. 1. On the other hand, the query sequence shows only an identity of 30.6% with the known Dermanyssus gallinae-derived cathepsin L (DgCatL-1, GenBank Accession No. CCC33064.1).

From the above, a protein consisting of the amino acid sequence set forth in SEQ ID NO. 1 (DgCatL-2) was estimated to be a novel protein, which is different from the known Dermanyssus gallinae-derived DgCatL-1.

### <Example 2> Cloning of DgFER2

### 1) Determination of the full-length DgFER2

Routine procedures were used to synthesize cDNAs from the mRNA fraction extracted from mite bodies of Dermanyssus gallinae . PCR was performed using cDNAs as a template and primers F2 (SEQ ID NO. 14) and R2 (SEQ ID NO. 15) designed by reference to the base sequences of tick FER2 genes. Using the amplified fragment, 5' RACE and 3' RACE was performed with the above-mentioned kits from Life Technologies, and the resulting full-length sequence was subjected to sequencing. In addition, primers F1 and R1 represented by SEQ ID NOs. 16 and 17, respectively, was prepared based on the determined sequence, and PCR was performed using cDNAs as a template. Sequencing of the amplified DNA fragment determined a base sequence set forth in SEQ ID NO. 4, which encodes a protein having a signal sequence of 18 amino acids added at the N terminus of the amino acid sequence set forth in SEQ ID NO. 2.

### 2) Homology search

A homology search in GenBank was performed using the amino acid sequence (including the signal sequence) encoded by the determined base sequence as a query sequence . The results of the homology search revealed that Metaseiulus occidentalis ferritin 2 (Accession No. XP_003737398.1) has an identity of 59.0%, Haemaphysalis longicornis ferritin 2 (Accession No. BAN13552.1) has an identity of 48.2%, Ixodes ricinus ferritin 2 (AccessionNo. ACJ70653.1) has an identity of 49.5%, and Ixodes persulcatusferritin2 (Accession No.AGX01000.1) has an identity of 49.5%, respectively with the query sequence. From these results of the homology search, a protein consisting of the amino acid sequence set forth in SEQ ID NO. 2 was estimated to be Dermanyssus gallinae FER2. The result of an alignment of these five amino acid sequences is shown in Fig. 2.

### <Example 3> Production of DgCatL-2 and DgFER2

A DNA sequence consisting of a base sequence encoding the amino acid sequence of SEQ ID NO. 1 determined in Example 1 was incorporated into pCold-TF (TaKaRa), an expression vector for Escherichia coli cells. Also, a DNA sequence consisting of a base sequence encoding the amino acid sequence of SEQ ID NO. 2 determined in Example 2 was incorporated into pCold-TF (TaKaRa) . These genetic recombinations allow DgCatL-2 and DgFER2 without their signal sequences to be expressed as fusion proteins with an Escherichia coli chaperone, Trigger Factor (TF; a molecular weight of 48 kDa), which is a solubilizing tag.

After the expression of each recombinant genes was induced by cold shock, the fusion proteins were purified by affinity chromatography against the His tag sequence, and then subjected to SDS-PAGE. A photograph showing a gel after electrophoresis is shown in Fig. 3.

In addition, for DgCatL-2, a DNA sequence encoding the amino acid sequence ranging from amino acid 314 to the C terminus of SEQ ID NO. 1 was incorporated into the above-described vector, pCold-TF, to express a fusion protein of a peptide fragment containing the active center site of DgCatL-2 and TF (Fig. 4) . The fusion protein was purified by affinity chromatography against the His tag sequence, and then subjected to SDS-PAGE. A photograph showing a gel after electrophoresis is shown in Fig. 4.

### <Example 4 > Expression of DgCatL-2 in Dermanyssus gallinae mites

Total RNA fractions were prepared from Dermanyssus gallinae mites collected in three different poultry farms. Primer set a of a forward primer consisting of the base sequence set forth in SEQ ID NO. 18 and a reverse primer consisting of the base sequence set forth in SEQ ID NO. 19, primer set b of a forward primer consisting of the base sequence set forth in SEQ ID NO. 20 and a reverse primer consisting of the base sequence set forth in SEQ ID NO. 21, and primer set c of a forward primer consisting of the base sequence set forth in SEQ ID NO. 22 and a reverse primer consisting of the base sequence set forth in SEQ ID NO. 23 were prepared to perform RT-PCR using the total RNAs as a template. The results of agarose gel electrophoresis of amplified fragments are shown in Fig. 5.

Amplified fragments of expected sizes were detected for each of three samples of Dermanyssus gallinae mites and for each of the primer sets a to c. In parallel, RT-PCR was performed in a similar way using total RNA from Ornithonyssus sylviarum mites collected as control, but no amplified fragment was detected.

### <Example 5> Vaccine administration test

### 1) Preparation of immunized plasma with recombinant protein

TF, and fusion proteins of TF and CatL-2 (TF-CatL2), TF andapeptide fragment containing the active center site of CatL-2 (TF-CatL2PD), and TF and DgFER2 (TF-FER2) which had been purified in Example 3 were dissolved in buffer to make their respective solutions of 0.25 mg/mL.

To 100 µL of each solutions was added the same volume of GERBU ADJUVANT (GERBU), and the mixture was intraperitoneally administered to 2-days old Boris Brown chickens (n = 2 to 4) at a dose of 200 µL per chicken. The day of the first immunization was defined as day 0. Two weeks after the first immunization, the chickens received an additional immunization in a similar way as in the first immunization, and were kept for another three weeks. The immunized chickens were euthanized by cardiac exsanguination under anesthesia. Heparin anticoagulated blood was centrifuged at 3,000 rpm for 10 minutes, and the resulting plasma was stored at -80°C. Separately, blood was collected from chickens which had not been sucked blood by Dermanyssus gallinae mites, subjected to anticoagulation treatment with heparin, centrifuged at 3, 000 rpm for 10 minutes, and the plasma was removed to obtain the blood cell component. The blood cell component and the plasma that had been prepared from the immunized chicken and stored were mixed to make a test blood.

In addition, the abdomen of chickens of the same variety was denuded of feathers, and pieces of epidermis were removed, immersed in ethanol, and stored at 4°C. After storage, the ethanol was replaced with PBS, the piece of epidermis was washed three times. Then, subcutaneous fat was removed from the piece of epidermis, which was then cut to a size of about 2 cm square with avoiding large pores, to prepare a chicken epidermis sheet.

### 2) In vitro feeding assay

Dermanyssus gallinae mites maintained under starvation conditions in a 50-mL tube for about 3 weeks were released into petri dishes on ice, and active mite individuals were transferred onto filter papers and then into 5-mL round tubes with cell strainer snap cap (Falcon) together with the respective filter papers. The number of Dermanyssus gallinae mites in each tubes was set to be from 10 to 20 individuals in a total of deutonymphs and adults. The opening of the round tube was covered with the chicken epidermis sheet prepared in the above section 1), onto which a 5-mL truncated syringe was placed.

2 mL of the test blood prepared in the above section 1) was warmed to 37 ° C, and then added into the syringe. The tube was stood on a rack in a slightly tilted state, and left to stand in a foamed polystyrene container. The foamed polystyrene container was placed in an 37°C-controlled incubator, so that the temperature and humidity in the container were maintained to be 35°C or higher and 70 to 90%, respectively. In this way, the mites were allowed to suck the test blood through the chicken epidermis sheet.

14 hours after the tube was left to stand, the test blood was discarded, and only the syringe was removed. The mites attached on the chicken epidermis sheet were made to drop by tapping the bottom of the tube, the chicken epidermis sheet was detached, and the tube was covered with a cell strainer snap cap. The number of mites of which the abdomen had turned red by bloodsucking was counted, and then the mites were maintained in the tube for another three weeks. The number of mites killed by bloodsucking was determined at the time points of weeks 1 and 3, and the mortality rate was calculated from the number of mites killed by bloodsucking, relative to the total number of mites having sucked the blood. The results are shown in Fig. 6.

3) A test of the effect of vaccines was carried out in a similar way as in the above sections 1) and 2), except that PNP/DO chickens were used in place of Boris Brown chickens. In this test, the number of dead mites, irrespective of whether they had sucked the blood or not, was determined daily over a period of 14 days after finishing of the bloodsucking. In addition, at the final day of experiment, 2 mL of ethanol was added into the tube, the mites were fixed, and the total number of mites in the tube was counted under a stereoscopic microscope. The mortality rate at the respective days was calculated from the number of dead mites, relative to the total number of mites. The results are shown in Fig. 7.

As shown in Figs. 6 and 7, it was revealed that the blood from the chickens immunized with each of the recombinant proteins had an activity to kill Dermanyssus gallinae mites, and that the blood from the chickens immunized with CatL-2 was more effective in killing Dermanyssus gallinae mites than that from the chickens immunized with DgFER2.

### Industrial Applicability

Since both DgCatL-2 and DgFER2 are factors involved in the digestion and storage of nutrients in redmites and inhibition of their functions would exerts great effects on their growth and propagation, it can be expected that DgCatL-2 , DgFER2, and/or immunological equivalents thereof find application as candidate antigens for new vaccines against red mites that have not been reported until now.

## Claims

1. A protein consisting of the amino acid sequence set forth in SEQ ID NO. 1, a protein consisting of the amino acid sequence set forth in SEQ ID NO. 2, and/or an immunological equivalent thereof, for use in eliciting an immune response specific for red mites in a domestic animal, wherein the immunological equivalent is selected from a group consisting of (b) to (j) described below and is capable of eliciting Dermanyssus gallinae-specific immune response;
(b) a protein consisting of an amino acid sequence in which one to several amino acids in the amino acid sequence set forth in SEQ ID NO. 1 are deleted or substituted and having an identity of at least 80% or higher with the amino acid sequence set forth in SEQ ID NO. 1,
(c) a protein consisting of an amino acid sequence in which one to several amino acids are inserted in the amino acid sequence set forth in SEQ ID NO. land having an identity of at least 80% or higher with the amino acid sequence set forth in SEQ ID NO. 1,
(f) a protein consisting of an amino acid sequence in which one to several amino acids in the amino acid sequence set forth in SEQ ID NO. 2 are deleted or substituted and having an identity of at least 80% or higher with the amino acid sequence set forth in SEQ ID NO. 2,
(g) a protein consisting of an amino acid sequence in which one to several amino acids are inserted in the amino acid sequence set forth in SEQ ID NO. 2 and having an identity of at least 80% or higher with the amino acid sequence set forth in SEQ ID NO. 2,
(i) a polypeptide fragment of the proteins consisting of the amino acid sequence set forth in SEQ ID NO. 1 or SEQ ID No. 2, or the proteins of the above (b) to (g), wherein the polypeptide fragment has no less than 30 amino acid residues, and
(j) a protein consisting of an amino acid sequence in which an optional amino acid (s) is/are added at the N- and/or C-terminus of the amino acid sequence of SEQ ID No. 1 or SEQ ID No. 2, or any one of the amino acid sequence defined in the above (b) to (i) .

2. A protein consisting of the amino acid sequence of any of a) to d) described below, or a polypeptide fragment consisting of a partial amino acid sequence of the amino acid sequence of a) to c) described below, wherein the protein or the polypeptide fragment is capable of eliciting Dermanyssus gallinae-specific immune response and the polypeptide fragment has no less than 30 amino acid residues:
a) the amino acid sequence set forth in SEQ ID NO. 1,
b) an amino acid sequence in which one to several amino acids in the amino acid sequence set forth in SEQ ID NO. 1 are deleted or substituted and having an identity of at least 80% or higher with the amino acid sequence set forth in SEQ ID NO. 1,
c) an amino acid sequence in which one to several amino acids are inserted in the amino acid sequence set forth in SEQ ID NO. 1 and having an identity of at least 80% or higher with the amino acid sequence set forth in SEQ ID NO. 1,
d) an amino acid sequence in which an optional amino acid (s) is/are added at theN- and/or C-terminus of the amino acid sequence of any of a) to c) or a partial amino acid sequence thereof.

3. A protein consisting of the amino acid sequence of any of e) to h) described below, or a polypeptide fragment consisting of a partial amino acid sequence of the amino acid sequence of e) to g) described below, wherein the protein or the polypeptide fragment is capable of eliciting Dermanyssus gallinae-specific immune response and the polypeptide fragment has no less than 30 amino acid residues:
e) the amino acid sequence set forth in SEQ ID NO. 2,
f) an amino acid sequence in which one to several amino acids in the amino acid sequence set forth in SEQ ID NO. 2 are deleted or substituted and having an identity of at least 80% or higher with the amino acid sequence set forth in SEQ ID NO. 2,
g) an amino acid sequence in which one to several amino acids are inserted in the amino acid sequence set forth in SEQ ID NO. 2 and having an identity of at least 80% or higher with the amino acid sequence set forth in SEQ ID NO. 2,
h) an amino acid sequence in which an optional amino acid (s) is/are added at theN- and/or C-terminus of the amino acid sequence of any of e) to g) or a partial amino acid sequence thereof.

4. A nucleic acid encoding the protein or the polypeptide fragment thereof as defined in claim 2 or claim 3.

5. An expression vector comprising the nucleic acid according to claim 4.

6. A vaccine for use in eliciting an immune response specific for red mites in a domestic animal, comprising the protein or the polypeptide fragment as defined in claim 2 and/or claim 3, or the nucleic acid as defined in claim 4 and/or the expression vector as defined in claim 5.

7. Proteins selected from the group consisting of a protein consisting of the amino acid sequence set forth in SEQ ID NO. 1, a protein consisting of the amino acid sequence set forth in SEQ ID NO. 2, and immunological equivalents thereof, for use in eliciting an immune response specific for red mites in a domestic animal, wherein the immunological equivalent is selected from a group consisting of (b) to (j) described below and is capable of eliciting Dermanyssus gallinae-specif ic immune response;
(b) a protein consisting of an amino acid sequence in which one to several amino acids in the amino acid sequence set forth in SEQ ID NO. 1 are deleted or substituted and having an identity of at least 80% or higher with the amino acid sequence set forth in SEQ ID NO. 1,
(c) a protein consisting of an amino acid sequence in which one to several amino acids are inserted in the amino acid sequence set forth in SEQ ID NO. land having an identity of at least 80% or higher with the amino acid sequence set forth in SEQ ID NO. 1,
(f) a protein consisting of an amino acid sequence in which one to several amino acids in the amino acid sequence set forth in SEQ ID NO. 2 are deleted or substituted and having an identity of at least 80% or higher with the amino acid sequence set forth in SEQ ID NO. 2,
(g) a protein consisting of an amino acid sequence in which one to several amino acids are inserted in the amino acid sequence set forth in SEQ ID NO. 2 and having an identity of at least 80% or higher with the amino acid sequence set forth in SEQ ID NO. 2,
(i) a polypeptide fragment of the proteins consisting of the amino acid sequence set forth in SEQ ID NO. 1 or SEQ ID No. 2, or the proteins of the above (b) to (g), wherein the polypeptide fragment has no less than 30 amino acid residues, and
(j) a protein consisting of an amino acid sequence in which an optional amino acid (s) is/are added at the N- and/or C-terminus of the amino acid sequence of SEQ ID No. 1 or SEQ ID No. 2, or any one of the amino acid sequence defined in the above (b) to (i) .

## Patentansprüche

1. Protein, bestehend aus der in SEQ ID NO. 1 dargestellten Aminosäuresequenz, Protein, bestehend aus der in SEQ ID NO. 2 dargestellten Aminosäuresequenz, und/oder ein immunologisches Äquivalent davon, zur Verwendung beim Hervorrufen einer Immunantwort, die spezifisch für Rote Vogelmilben in einem Haustier ist, wobei das immunologische Äquivalent ausgewählt ist aus einer unten beschriebenen Gruppe bestehend aus (b) bis (j), und in der Lage ist, eine Dermanyssus-gallinae-spezifische Immunantwort hervorzurufen;
(b) Protein, bestehend aus einer Aminosäuresequenz, in der eine bis mehrere Aminosäuren in der in SEQ ID NO. 1 dargestellten Aminosäuresequenz deletiert oder substituiert sind und eine Identität von mindestens 80% oder höher mit der in SEQ ID NO. 1 dargestellten Aminosäuresequenz aufweisen,
(c) Protein, bestehend aus einer Aminosäuresequenz, in die eine bis mehrere Aminosäuren in die in SEQ ID NO. 1 dargestellte Aminosäuresequenz eingefügt sind und das eine Identität von mindestens 80% oder höher mit der in SEQ ID NO. 1 dargestellten Aminosäuresequenz aufweist,
(f) Protein, bestehend aus einer Aminosäuresequenz, in der eine bis mehrere Aminosäuren in der in SEQ ID NO. 2 dargestellten Aminosäuresequenz deletiert oder substituiert sind und eine Identität von mindestens 80% oder höher mit der in SEQ ID NO. 2 dargestellten Aminosäuresequenz aufweisen,
(g) Protein, bestehend aus einer Aminosäuresequenz, in die eine bis mehrere Aminosäuren in die in SEQ ID NO. 2 dargestellte Aminosäuresequenz eingefügt sind und das eine Identität von mindestens 80% oder höher mit der in SEQ ID NO. 2 dargestellten Aminosäuresequenz aufweist,
(i) Polypeptidfragment der Proteine, bestehend aus der in SEQ ID NO. 1 oder SEQ ID NO. 2 dargestellten Aminosäuresequenz oder den Proteinen der vorstehenden (b) bis (g), wobei das Polypeptidfragment nicht weniger als 30 Aminosäurereste aufweist, und
(j) Protein, bestehend aus einer Aminosäuresequenz, in der eine oder mehrere optionale Aminosäure(n) am N- und/oder C-Terminus der Aminosäuresequenz von SEQ ID Nr. 1 oder SEQ ID Nr. 2 oder irgendeiner der in den vorstehenden (b) bis (i) definierten Aminosäuresequenzen hinzugefügt wird/werden.

2. Protein, bestehend aus der Aminosäuresequenz von irgendeinem a) bis d) wie nachstehend beschrieben, oder Polypeptidfragment, bestehend aus einer partiellen Aminosäuresequenz der Aminosäuresequenz von a) bis c) wie nachstehend beschrieben, wobei das Protein oder das Polypeptidfragment in der Lage ist, eine Dermanyssus-gallinae-spezifische Immunantwort hervorzurufen, und wobei das Polypeptidfragment nicht weniger als 30 Aminosäurereste aufweist:
a) die in SEQ ID NO. 1 dargestellte Aminosäuresequenz,
b) Aminosäuresequenz, in der eine bis mehrere Aminosäuren in der in SEQ ID NO. 1 dargestellten Aminosäuresequenz deletiert oder substituiert sind und eine Identität von mindestens 80% oder höher mit der in SEQ ID NO. 1 dargestellten Aminosäuresequenz aufweisen,
c) Aminosäuresequenz, in die eine bis mehrere Aminosäuren in die in SEQ ID NO. 1 dargestellte Aminosäuresequenz eingefügt sind und die eine Identität von mindestens 80% oder höher mit der in SEQ ID NO. 1 dargestellten Aminosäuresequenz aufweist,
d) Aminosäuresequenz, in der eine optionale Aminosäure(n) am N- und/oder C-Terminus der Aminosäuresequenz von irgendeinem a) bis c) oder einer partiellen Aminosäuresequenz davon hinzugefügt wird/sind.

3. Protein, bestehend aus der Aminosäuresequenz von irgendeinem e) bis h) wie nachstehend beschrieben oder Polypeptidfragment, bestehend aus einer partiellen Aminosäuresequenz der Aminosäuresequenz von e) bis g) wie nachstehend beschrieben, wobei das Protein oder das Polypeptidfragment in der Lage ist, eine Dermanyssus-gallinae-spezifische Immunantwort hervorzurufen und das Polypeptidfragment nicht weniger als 30 Aminosäurereste aufweist:
e) die in SEQ ID NO, 2 dargestellte Aminosäuresequenz,
f) Aminosäuresequenz, in der eine bis mehrere Aminosäuren in der in SEQ ID NO. 2 dargestellten Aminosäuresequenz deletiert oder substituiert sind und eine Identität von mindestens 80% oder höher mit der in SEQ ID NO. 2 dargestellten Aminosäuresequenz aufweisen,
g) Aminosäuresequenz, in die eine bis mehrere Aminosäuren in die in SEQ ID NO. 2 dargestellte Aminosäuresequenz eingefügt sind und die eine Identität von mindestens 80% oder höher mit der in SEQ ID NO. 2 dargestellten Aminosäuresequenz aufweist,
h) eine Aminosäuresequenz, in der eine oder mehrere optionale Aminosäure(n) am N- und/oder C-Terminus der Aminosäuresequenz von irgendeinem e) bis g) oder einer partiellen Aminosäuresequenz davon hinzugefügt wird/werden.

4. Nukleinsäure, die das Protein oder das Polypeptidfragment davon wie in Anspruch 2 oder Anspruch 3 definiert, kodiert.

5. Expressionsvektor, umfassend die Nukleinsäure nach Anspruch 4.

6. Impfstoff zur Verwendung beim Hervorrufen einer Immunantwort, die spezifisch für Rote Vogelmilben in einem Haustier ist, umfassend das Protein oder das Polypeptidfragment wie in Anspruch 2 und/oder Anspruch 3 definiert, oder die Nukleinsäure wie in Anspruch 4 definiert, und/oder den Expressionsvektor wie in Anspruch 5 definiert.

7. Proteine, ausgewählt aus der Gruppe bestehend aus einem Protein, bestehend aus der in SEQ ID NO. 1 dargestellten Aminosäuresequenz, einem Protein, bestehend aus der in SEQ ID NO. 2 dargestellten Aminosäuresequenz, und immunologischen Äquivalenten davon, zur Verwendung beim Hervorrufen einer Immunantwort, die spezifisch für Rote Milben in einem Haustier ist, wobei das immunologische Äquivalent ausgewählt ist aus einer Gruppe bestehend aus den nachstehend beschriebenen (b) bis (j), und in der Lage ist, eine Dermanyssus-gallinae-spezifische Immunantwort auszulösen;
(b) Protein, bestehend aus einer Aminosäuresequenz, in der eine bis mehrere Aminosäuren in der in SEQ ID NO. 1 dargestellten Aminosäuresequenz deletiert oder substituiert sind und eine Identität von mindestens 80% oder höher mit der in SEQ ID NO. 1 dargestellten Aminosäuresequenz aufweisen,
(c) Protein, bestehend aus einer Aminosäuresequenz, in die eine bis mehrere Aminosäuren in die in SEQ ID NO. 1 dargestellte Aminosäuresequenz eingefügt sind und das eine Identität von mindestens 80% oder höher mit der in SEQ ID NO. 1 dargestellten Aminosäuresequenz aufweist,
(f) Protein, bestehend aus einer Aminosäuresequenz, in der eine bis mehrere Aminosäuren in der in SEQ ID NO. 2 dargestellten Aminosäuresequenz deletiert oder substituiert sind und eine Identität von mindestens 80% oder höher mit der in SEQ ID NO. 2 dargestellten Aminosäuresequenz aufweisen,
(g) Protein, bestehend aus einer Aminosäuresequenz, in die eine bis mehrere Aminosäuren in die in SEQ ID NO. 2 dargestellte Aminosäuresequenz eingefügt sind und das eine Identität von mindestens 80% oder höher mit der in SEQ ID NO. 2 dargestellten Aminosäuresequenz aufweist,
(i) Polypeptidfragment der Proteine, bestehend aus der in SEQ ID NO. 1 oder SEQ ID NO. 2 dargestellten Aminosäuresequenz oder den Proteinen der vorstehenden (b) bis (g), wobei das Polypeptidfragment nicht weniger als 30 Aminosäurereste aufweist, und
(j) Protein, bestehend aus einer Aminosäuresequenz, in der eine optionale Aminosäure(n) am N- und/oder C-Terminus der Aminosäuresequenz von SEQ ID NO. 1 oder SEQ ID NO. 2 oder irgendeiner der in den vorstehenden (b) bis (i) definierten Aminosäuresequenzen hinzugefügt wird/werden.

## Revendications

1. Protéine constituée par la séquence d'acides aminés présentée dans SEQ ID N°: 1, protéine constituée par la séquence d'acides aminés présentée dans SEQ ID N°: 2, et équivalent immunologique de celles-ci, pour une utilisation dans l'obtention d'une réponse immunitaire spécifique pour des poux rouges chez un animal domestique, dans lesquels l'équivalent immunologique est sélectionné à partir d'un groupe constitué par les (b) à (j) décrits ci-dessous et est capable d'obtenir une réponse immunitaire spécifique à Dermanyssus gallinae ;
(b) protéine constituée par une séquence d'acides aminés dans laquelle un à plusieurs acides aminés dans la séquence d'acides aminés présentée dans SEQ ID N°: 1 sont délétés ou substitués et ayant une identité d'au moins 80% ou plus avec la séquence d'acides aminés présentée dans SEQ ID N°: 1,
(c) protéine constituée par une séquence d'acides aminés dans laquelle un à plusieurs acides aminés sont insérés dans la séquence d'acides aminés présentée dans SEQ ID N°: 1 et ayant une identité d'au moins 80% ou plus avec la séquence d'acides aminés présentée dans SEQ ID N°: 1,
(f) protéine constituée par une séquence d'acides aminés dans laquelle un à plusieurs acides aminés dans la séquence d'acides aminés présentée dans SEQ ID N°: 2 sont délétés ou substitués et ayant une identité d'au moins 80% avec la séquence d'acides aminés présentée dans SEQ ID N°: 2,
(g) protéine constituée par une séquence d'acides aminés dans laquelle un à plusieurs acides aminés sont insérés dans la séquence d'acides aminés présentée dans SEQ ID N°: 2 et ayant une identité d'au moins 80% ou plus avec la séquence d'acides aminés présentée dans SEQ ID N°: 2,
(i) fragment polypeptidique des protéines constitué par la séquence d'acides aminés présentée dans SEQ ID N°: 1 ou SEQ ID N°: 2, ou protéines des (b) à (g) ci-dessus, dans lesquels le fragment polypeptidique comporte pas moins de 30 résidus d'acides aminés, et
(j) protéine constituée par une séquence d'acides aminés dans laquelle un ou des acides aminés facultatifs sont ajoutés à la terminaison N et/ou C de la séquence d'acides aminés de SEQ ID N°: 1 ou SEQ ID N°: 2, ou l'une quelconque de la séquence d'acides aminés définie dans les (b) à (i) ci-dessus.

2. Protéine constituée par la séquence d'acides aminés de l'un quelconque des a) à d) décrits ci-dessous, ou fragment polypeptidique constitué par une séquence d'acides aminés partielle de la séquence d'acides aminés des a) à c) décrits ci-dessous, dans lesquels la protéine ou le fragment polypeptidique est capable d'obtenir une réponse immunitaire spécifique à Dermanyssus gallinae et le fragment polypeptidique comporte pas moins de 30 résidus d'acides aminés :
a) la séquence d'acides aminés présentée dans SEQ ID N°: 1,
b) une séquence d'acides aminés dans laquelle un à plusieurs acides aminés dans la séquence d'acides aminés présentée dans SEQ ID N°: 1 sont délétés ou substitués et ayant une identité d'au moins 80% ou plus avec la séquence d'acides aminés présentée dans SEQ ID N°: 1,
c) une séquence d'acides aminés dans laquelle un à plusieurs acides aminés sont insérés dans la séquence d'acides aminés présentée dans SEQ ID N°: 1 et ayant une identité d'au moins 80 % ou plus avec la séquence d'acides aminés présentée dans SEQ ID N°: 1,
d) une séquence d'acides aminés dans laquelle un ou des acides aminés facultatifs sont ajoutés dans la terminaison N et/ou C de la séquence d'acides aminés de l'un quelconque des a) à c) ou d'une séquence d'acides aminés partielle de celle-ci.

3. Protéine constituée par la séquence d'acides aminés de l'un quelconque des e) à h) décrits ci-dessous, ou fragment polypeptidique constitué par une séquence d'acides aminés partielle de la séquence d'acides aminés des e) à g) décrits ci-dessous, dans lesquels la protéine ou le fragment polypeptidique est capable d'obtenir une réponse immunitaire spécifique à Dermanyssus gallinae et le fragment polypeptidique comporte pas moins de 30 résidus d'acides aminés :
e) la séquence d'acides aminés présentée dans SEQ ID N°: 2,
f) une séquence d'acides aminés dans laquelle un à plusieurs acides aminés dans la séquence d'acides aminés présentée dans SEQ ID N°: 2 sont délétés ou substitués et ayant une identité d'au moins 80% ou plus avec la séquence d'acides aminés présentée dans SEQ ID N°: 2,
g) une séquence d'acides aminés dans laquelle un à plusieurs acides aminés sont insérés dans la séquence d'acides aminés présentée dans SEQ ID N°: 2 et ayant une identité d'au moins 80 % ou plus avec la séquence d'acides aminés présentée dans SEQ ID N°: 2,
h) une séquence d'acides aminés dans laquelle un ou des acides aminés facultatifs sont ajoutés au niveau de la terminaison N et/ou C de la séquence d'acides aminés de l'un quelconque des e) à g) ou d'une séquence d'acides aminés partielle de celle-ci.

4. Acide nucléique codant pour la protéine ou le fragment polypeptidique de celle-ci selon la revendication 2 ou la revendication 3.

5. Vecteur d'expression comprenant l'acide nucléique selon la revendication 4.

6. Vaccin pour une utilisation dans l'obtention d'une réponse immunitaire spécifique pour des poux rouges chez un animal domestique, comprenant la protéine ou le fragment polypeptidique selon la revendication 2 et/ou la revendication 3, ou l'acide nucléique selon la revendication 4 et/ou le vecteur d'expression selon la revendication 5.

7. Protéines sélectionnées à partir du groupe constitué par une protéine constituée par la séquence d'acides aminés présentée dans SEQ ID N°: 1, une protéine constituée par la séquence d'acides aminés présentée dans SEQ ID N°: 2 et des équivalents biologiques de celles-ci, pour une utilisation dans l'obtention d'une réponse immunitaire spécifique pour des poux rouges chez un animal domestique, dans lesquelles, l'équivalent immunologique est sélectionné à partir d'un groupe constitué par les (b) à (j) décrits ci-dessous et est capable d'obtenir une réponse immunitaire spécifique à Dermanyssus gallinae ;
(b) une protéine constituée par une séquence d'acides aminés dans laquelle un à plusieurs acides aminés dans la séquence d'acides aminés présentée dans SEQ ID N°: 1 sont délétés ou substitués et ayant une identité d'au moins 80% ou plus avec la séquence d'acides aminés présentée dans SEQ ID N°: 1,
(c) une protéine constituée par une séquence d'acides aminés dans laquelle un à plusieurs acides aminés sont insérés dans la séquence d'acides aminés présentée dans SEQ ID N°: 1 et ayant une identité d'au moins 80% ou plus avec la séquence d'acides aminés présentée dans SEQ ID N°: 1,
(f) une protéiné constituée par une séquence d'acides aminés dans laquelle un à plusieurs acides aminés dans la séquence d'acide aminés présentée dans SEQ ID N°: 2 sont délétés ou substitués et ayant une identité d'au moins 80% avec la séquence d'acides aminés présentée dans SEQ ID N°: 2,
(g) une protéine constituée par une séquence d'acides aminés dans laquelle un à plusieurs acides aminés sont insérés dans la séquence d'acides aminés présentée dans SEQ ID N°: 2 et ayant une identité d'au moins 80% ou plus avec la séquence d'acides aminés présentée dans SEQ ID N°:2,
(i) un fragment polypeptidique des protéines constituées par la séquence d'acides aminés présentée dans SEQ ID N°: 1 ou SEQ ID N°: 2, ou les protéines des (b) à (g) ci-dessus, dans lesquelles le fragment polypeptidique comporte pas moins de 30 résidus d'acides aminés, et
(j) une protéine constituée par une séquence d'acides aminés dans laquelle un ou des acides aminés facultatifs sont ajoutés au niveau de la terminaison N et/ou C de la séquence d'acides aminés de SEQ ID N°: 1 ou SEQ ID N°: 2, ou l'une quelconque de la séquence d'acides aminés définie dans les (b) à (i) ci-dessus.


### SEQUENCE LISTING

<110> National University Corporation Hokkaido University
<120> Method for controlling red mite and proteins used therein
<130> P14014WO
<160> 25
<170> PatentIn version 3.5
<210> 1
   <211> 531
   <212> PRT
   <213> Dermanyssus gallinae
<400> 1
   Val Ser Val Pro Arg Gly Ala Pro Glu Phe Pro Pro Ser Tyr Thr Ala
   1               5                   10                  15
   Ser Gly Tyr Ile Leu Leu Pro Tyr Cys Glu Leu Arg Glu Pro Phe Thr
               20                  25                  30
   Ala Tyr Tyr Asp Gly Glu Ser Asp Arg Ser Arg Ile Asp Tyr Tyr Asp
           35                  40                  45
   Gly Glu Met Lys Thr Phe Val Gly Lys Ser Gly Thr Phe Lys Val Val
       50                  55                  60
   Trp Ser Pro Asn Glu Lys Thr His Ile Pro Glu Leu Asn Cys Tyr Glu
   65                  70                  75                  80
   Ala Gly Pro Ala Lys Ser Gln Ser Ile Leu Pro Asp Leu Thr Asn Phe
                   85                  90                  95
   Thr Phe Val Arg Val Glu Pro Cys Glu Thr Asp Ser Thr His Ile Val
               100                 105                 110
   Lys Pro Leu Leu Arg Gly Ala Asp Lys Cys Tyr Arg Tyr Glu Lys Lys
           115                 120                 125
   Val Asp Asn Phe Gly Arg Val Ser Lys Tyr Thr Phe Trp Ala Ser Gln
       130                 135                 140
   Asp Asp Asp Asn Thr Pro Ile Pro Val Arg Tyr Val Met Met Gly Tyr
   145                 150                 155                 160
   Asp Ser Leu Leu Gly Ser His Phe Asp Lys Tyr Glu Val Val Tyr Thr
                   165                 170                 175
   Asp Tyr Thr Pro Gly Pro Val Glu Asp Asp Leu Phe Gln Val Lys Thr
               180                 185                 190
   Val Ile Asp Lys Glu Cys Thr Ser Phe Pro Ser Pro Pro Gly Val Ser
           195                 200                 205
   Thr Thr His Leu Phe Asn Pro Met Ala Glu Phe Ile Asp Glu Lys Asp
       210                 215                 220
   Ser His Val His Glu His Phe Glu His Phe Lys Ser Thr His Gly Lys
   225                 230                 235                 240
   Ala Tyr Gly His Gln Ala Glu Glu Ile Ile Arg Lys Asp Asn Phe Arg
                   245                 250                 255
   His Asn Gln Arg Phe Val Asn Ser Met Asn Arg Arg Asn Leu Ser Tyr
               260                 265                 270
   Ala Leu Lys Leu Asn His Arg Ala Asp Trp Ser Gln Asp Glu Phe Arg
           275                 280                 285
   Leu Leu Arg Gly Arg Leu Gln Phe Thr Ser Gln Lys Ser Met Ala Arg
       290                 295                 300
   Glu Phe Pro Lys Glu Gln Tyr Ser Asp Arg Val Glu Pro Asp Tyr Val
   305                 310                 315                 320
   Asp Trp Arg Leu Glu Gly Ala Val Thr Pro Val Lys Asp Gln Ala Val
                   325                 330                 335
   Cys Gly Ser Cys Trp Ser Phe Gly Thr Val Gly His Ile Glu Gly Ala
               340                 345                 350
   Tyr Phe Arg Lys Phe Gly Glu Leu Val Arg Phe Ser Glu Gln Gln Leu
           355                 360                 365
   Val Asp Cys Ser Trp Asn Ala Gly Asn Asp Ala Cys Asp Gly Gly Leu
       370                 375                 380
   Asp Phe Ile Ala Tyr His Tyr Ile Gln Lys Tyr Gly Leu Ala Ser Asn
   385                 390                 395                 400
   Asp Gln Tyr Gly Pro Tyr Arg Gly Ile Asp Gly Lys Cys Lys Asp Leu
                   405                 410                 415
   Glu Ile Ser Asn Lys Pro Ile Ser Thr Leu Lys Gly Tyr Arg Asn Val
               420                 425                 430
   Thr Thr Val Glu Asp Leu Arg Lys Ala Leu Ala Phe Val Gly Pro Ile
           435                 440                 445
   Ser Val Ser Ile Asp Ala Ser Arg Pro Ser Leu Ser Phe Tyr Ser His
       450                 455                 460
   Gly Val Tyr Ser Asp Pro Asp Cys Ser Ser Thr Glu Leu Asp His Ser
   465                 470                 475                 480
   Val Leu Ala Val Gly Tyr Gly Thr Leu His Gly Glu Pro Tyr Trp Leu
                   485                 490                 495
   Ile Lys Asn Ser Trp Ser Thr Tyr Trp Gly Asn Asp Gly Tyr Ile Leu
               500                 505                 510
   Ile Ser Gln Lys Asp Asn Met Cys Gly Val Ala Ser Gln Ala Thr Tyr
           515                 520                 525
   Val Glu Leu
       530
<210> 2
   <211> 178
   <212> PRT
   <213> Dermanyssus gallinae
<400> 2
   Arg Gln Asp Asn Ala Thr Pro Ser Gly Asn Thr Asn Lys Tyr Val Leu
   1               5                   10                  15
   Glu Asp Ser Cys Leu Gly Gly Leu Arg Ala Gln Leu Asn Leu Glu Met
               20                  25                  30
   His Ala Ser Leu Leu Tyr Gln Gln Met Ala Ala His Phe Asp Ser Asn
           35                  40                  45
   Gln Val Ala His Lys Gly Phe Ala Lys Phe Phe Glu Lys Ser Ser Asp
       50                  55                  60
   Glu Glu Arg Glu His Ala Lys Lys Ile Thr Asn Tyr Ile Asn Ala Arg
   65                  70                  75                  80
   Gly Gly Thr Ile Gly His Leu Asn Val Arg Met Pro Ser Ser Asn Ser
                   85                  90                  95
   Trp Asp Thr Ala Lys Glu Ala Leu Gln Ala Ala Leu Val Leu Glu His
               100                 105                 110
   His Val Asn Asn Glu Leu His Leu Leu His Arg Thr Ala Asp Glu Asp
           115                 120                 125
   Cys Arg Asp Pro Gln Leu Gln Asp Phe Leu Glu Ser Asn Phe Leu Ser
       130                 135                 140
   Glu Gln Val Glu Ser Ile Ala Gln Ile Glu Arg Leu Ile Thr Asn Leu
   145                 150                 155                 160
   Asn Lys Phe Gly Asp Val His Leu Gly Glu Tyr Phe Val Asn Lys Asp
                   165                 170                 175
   Leu Leu
<210> 3
   <211> 1650
   <212> DNA
   <213> Dermanyssus gallinae
<400> 3
   atgttgatcc gctgcgttgt cacggctctt gcagccgtga cggttgtctc aggagtttcg      60
   gtgccccgag gtgcgccaga gttcccgccg tcctacactg cgtcgggcta catcctcctg     120
   ccgtactgtg agctgcgaga gccgttcacc gcctactacg atggtgaatc ggaccgatcg     180
   cgtattgact attacgacgg cgagatgaag acattcgtcg gaaaatcagg cacgtttaag     240
   gttgtctggt cgcccaatga gaagacacat ataccggagc tgaactgcta cgaggccgga     300
   ccggctaaaa gccagagcat tttgccggac ctcaccaact tcaccttcgt tcgggtcgag     360
   ccgtgcgaaa ccgattcgac gcacattgtc aagcccctgc tccgaggtgc tgacaaatgc     420
   tatcgctatg agaagaaagt ggataacttc ggacgcgtct ccaagtacac gttctgggcc     480
   tcacaggacg acgataacac cccaattccg gtacgctacg tcatgatggg ttacgactca     540
   ctcttgggat cgcacttcga caagtatgag gtcgtgtaca ccgactacac acccggaccc     600
   gtcgaagatg acctcttcca agtcaagact gttattgaca aggaatgcac ttcgttcccg     660
   tcgccgccgg gcgtgtccac tacccacctg ttcaacccga tggcggagtt cattgacgag     720
   aaggattcgc acgtgcacga acacttcgag cacttcaaat cgacacacgg caaggcatac     780
   ggccaccagg ccgaggagat catccgcaag gacaatttcc gccacaacca acgcttcgtc     840
   aattcgatga accgccgcaa cctttcgtac gcgctgaagc tcaaccaccg cgccgactgg     900
   agccaggacg agttcaggct gctccggggc cgtctacagt tcaccagcca gaagtcgatg     960
   gccagggagt tccccaagga acagtactcg gatcgcgtcg agccggacta cgtcgactgg    1020
   cgactcgagg gagccgtcac gccggtcaag gaccaggctg tgtgcgggtc gtgctggagc    1080
   tttggcacgg tcggccacat cgaaggcgcc tacttccgca agttcggcga gctggtccgt    1140
   ttctccgagc agcagctcgt agactgttcg tggaatgccg gcaacgatgc ctgcgacggt    1200
   ggtctggact ttatcgccta ccactacatc cagaagtacg gactggccag caacgaccaa    1260
   tacggaccct accgcggcat tgacggcaaa tgcaaggacc tggagatttc caacaagccc    1320
   attagcacgc tgaaaggcta ccgaaacgtg accactgtgg aagacctccg caaggcgctc    1380
   gcgtttgtcg gccccatatc ggtgtcgatc gatgcatcaa ggccgtcgct cagcttctat    1440
   tcgcatggag tctacagcga tccggactgc agttcgacgg aactcgacca ctccgtgctc    1500
   gctgttggct acggcacgct gcacggtgag ccgtactggc tcatcaagaa ctcgtggtcc    1560
   acgtactggg gcaacgacgg atacattctc atctcgcaga aggacaacat gtgcggcgtt    1620
   gcctcgcagg caacctacgt cgagctgtag                                     1650
<210> 4
   <211> 591
   <212> DNA
   <213> Dermanyssus gallinae
<400> 4
   atgaaacgtc tcgtctgcat cctcgtaccg ctgctcttcg cagctgtgct cgctcgccaa    60
   gataatgcca ccccatcggg caatacgaac aaatacgtgc tcgaggacag ctgtctgggg   120
   ggcttgcgag cccagttaaa cctcgagatg cacgcctcgc tcctctacca acagatggcc   180
   gcacactttg actccaacca ggttgcccac aagggcttcg ctaagttctt cgagaagagt   240
   tcggacgagg agcgtgagca cgcgaagaaa atcaccaact acattaacgc acgtggcggc   300
   accatcggcc atctcaacgt acggatgccg agctccaact catgggatac ggcaaaagag   360
   gcccttcaag ccgccctagt cctcgagcat cacgtcaaca acgagttgca ccttctccac   420
   agaacggccg acgaggactg tcgtgatccc cagttgcagg acttcttaga gagcaacttc   480
   ctcagcgagc aggtggaatc catcgctcag attgagcgtt tgataacgaa cctcaacaag   540
   ttcggtgatg tgcatctcgg agaatacttt gtcaacaagg accttctcta g            591
<210> 5
   <211> 553
   <212> PRT
   <213> Metaseiulus occidentalis
<400> 5
   Met Leu Leu Arg Val Ile Pro Val Val Ala Leu Ala Ile Val Ser Val
   1               5                   10                  15
   Ala Gly Gly Pro Ala Asn Pro Arg Asp Ala Pro Val Phe Pro Pro Ala
               20                  25                  30
   Tyr Ala Ala Ser Gly Tyr Ile Leu Leu Pro Tyr Cys Glu Leu Arg Glu
           35                  40                  45
   Pro Phe Thr Ala Tyr Tyr Asp Lys Thr Ala Gly Lys Ser Arg Ile Asp
       50                  55                  60
   Tyr Tyr Gly Gly Glu Met Lys Thr Phe Gln Ile Ala Gly Glu Thr Gly
   65                  70                  75                  80
   Gly Ser Tyr Lys Val Val Trp Ser Pro Asn Asn Glu Thr His Val Pro
                   85                  90                  95
   Glu Gln Asn Cys Tyr Ala Ala Gly Pro Ala Ala Pro Gln Gly Ile Leu
               100                 105                 110
   Pro Asp Leu Ser Asp Phe Lys Phe Val Arg Thr Glu Ser Cys Phe Thr
           115                 120                 125
   Asp Thr Ser Thr Leu Leu Asn Thr Leu Leu Ser Asn Ala Ala Lys Cys
       130                 135                 140
   Tyr Arg Tyr Glu Asn Thr Val Gln Lys Tyr Asp Arg Val Ser Lys Tyr
   145                 150                 155                 160
   Val Phe Trp Ala Asn Gln Asp Gly Asp Asn Asn Val Ile Pro Val Arg
                   165                 170                 175
   Tyr Met Met Met Gly Tyr Asp Ser Leu Leu Gly Ser His Phe Asp Lys
               180                 185                 190
   Tyr Glu Val Ile Tyr Asp Asp Tyr Thr Pro Gly Pro Val Asp Gly Asp
           195                 200                 205
   Val Phe Asp Val Thr Ser Val Ile Asp Lys Pro Cys Thr Asp Phe Pro
       210                 215                 220
   Ser Pro Pro Gly Gln Ser Val Ser His Leu Phe Asn Pro Met Lys Glu
   225                 230                 235                 240
   Phe Val His His Asp Asp Ser His Val Asp Glu His Phe Ser Asn Phe
                   245                 250                 255
   Lys Asn Glu His Gly Lys Ser Tyr Glu His Pro Thr Glu Glu Arg Glu
               260                 265                 270
   Arg Arg His Asn Phe His His Asn Met Arg Phe Val Asn Ser Met Asn
           275                 280                 285
   Arg Arg Asn Leu Ser Phe Ala Leu Lys Leu Asn Asn Arg Ala Asp Trp
       290                 295                 300
   Asn Gln Gly Glu Phe Lys Leu Leu Arg Gly Arg Leu Gln Ser Thr Asn
   305                 310                 315                 320
   Val Lys Ser Ser Ala Glu Asp Phe Pro Lys Glu Lys Phe Glu His Arg
                   325                 330                 335
   Thr Val Pro Asp Tyr Val Asp Trp Arg Leu Glu Gly Ala Val Thr Pro
               340                 345                 350
   Val Lys Asp Gln Ala Ile Cys Gly Ser Cys Trp Ser Phe Gly Thr Val
           355                 360                 365
   Gly His Ile Glu Gly Gln Tyr Phe Leu Lys His Gly Glu Leu Val Arg
       370                 375                 380
   Phe Ala Glu Gln Gln Leu Val Asp Cys Ser Trp Thr Ser Gly Asn Asp
   385                 390                 395                 400
   Ala Cys Asp Gly Gly Leu Asp Tyr Val Ala Tyr Asp Tyr Ile Lys Lys
                   405                 410                 415
   Tyr Gly Leu Ser Ser Asp Ala Gln Tyr Gly Pro Tyr Arg Gly Ile Asp
               420                 425                 430
   Gly Lys Cys Lys Asp Val Glu Ile Glu Asn Lys Pro Ile Thr Thr Ile
           435                 440                 445
   Gln Arg Tyr Tyr Asn Ile Ser Gly Val Glu Asn Leu Arg Lys Ala Ile
       450                 455                 460
   Ala Phe Val Gly Pro Ile Ser Val Ala Ile Asp Ala Ser Arg Pro Ser
   465                 470                 475                 480
   Leu Ser Phe Tyr Ala His Gly Val Tyr Glu Asp Pro Asp Cys Ser Ser
                   485                 490                 495
   Thr Glu Leu Asp His Ala Val Leu Ala Val Gly Tyr Gly Val Leu His
               500                 505                 510
   Gly Lys Pro Tyr Trp Leu Ile Lys Asn Ser Trp Ser Thr Tyr Trp Gly
           515                 520                 525
   Asn Asp Gly Tyr Ile Leu Ile Ser Gln Lys Asp Asn Met Cys Gly Val
       530                 535                 540
   Ala Ser Thr Pro Thr Tyr Val Glu Leu
   545 550
<210> 6
   <211> 564
   <212> PRT
   <213> Ixodes scapularis
<400> 6
   Met Ser Ser Val Leu Cys Gly Trp Leu Tyr Phe Ala Ala Leu Cys Leu
   1 5 10 15
   Gly Gln Glu Arg Pro Glu Trp Gly Asp Val Tyr Lys Val Lys Gly Val
   20 25 30
   Leu Tyr Leu Pro Tyr Ala Glu Ile Arg Glu Pro Phe Thr Gly Tyr Phe
   35 40 45
   Asp Ala Ser Gln Asn Leu Ser Arg Ile Asp Tyr Tyr Arg Gly Met Val
   50 55 60
   Gln Thr Ile Gln Val Ala Pro Asp Ala Glu Glu Gly Arg Ser Glu Thr
   65 70 75 80
   Pro Tyr Gly Ala Asn Tyr Lys Ile Ala Tyr Met Pro Asp Lys Glu Thr
   85 90 95
   Trp Gln Pro Arg Arg Thr Cys Phe Leu Val Asn Gly Thr Ala Asn Asn
   100 105 110
   Thr Val Pro Leu Gln Ala Val Leu Pro Asp Val Thr Ser Phe Glu Phe
   115 120 125
   Val Arg Gln Glu Ser Cys Trp Glu Gly Thr Thr Ala Val Asp Glu Pro
   130 135 140
   Ala Glu Arg Ala Ser Cys Glu Arg Phe Gln Leu Val Val Thr Asn Glu
   145 150 155 160
   Asn Arg Val Ser Lys Tyr Thr Leu Trp Val Ser Arg Asp Gly Gln Gly
   165 170 175
   Arg Ala Val Pro Arg Arg Tyr Leu Met Met Gly Tyr Asn Thr Leu Leu
   180 185 190
   Gly Ser His Phe Asp Lys Tyr Glu Leu Ile Tyr His Gly Phe Ser Arg
   195 200 205
   Lys Pro Leu Pro Ser Ser Val Phe Asp Ile Thr His Leu Ile Asn Glu
       210                 215                 220
   Thr Cys Arg Arg Phe Pro Gly Pro Gly Ala Glu His Leu Ala Leu His
   225                 230                 235                 240
   Ser Pro Met Ala Glu Phe Met Asn Gly His Asp Ala His Met His Ser
                   245                 250                 255
   Ala Phe Asp Lys Phe Arg Glu Asp His Ser Arg Asp Tyr Gly His His
               260                 265                 270
   Thr Glu His Glu Arg Arg Arg Asp Ile Phe Arg Gln Asn Leu Arg Phe
           275                 280                 285
   Ile His Ser Thr Asn Arg Ala Asn Arg Gly Tyr Thr Val Lys Val Asn
       290                 295                 300
   His Leu Ala Asp Arg Ser Ser Asp Glu Leu Gly Tyr Leu Arg Gly Arg
   305                 310                 315                 320
   Leu Gln Ser Arg Ala Pro Thr Asn Ala Leu Pro Phe Pro Lys Asp Arg
                   325                 330                 335
   Phe Ser Ser Asp Leu Pro Asp Tyr Val Asp Trp Arg Leu Tyr Gly Ala
               340                 345                 350
   Val Thr Pro Val Lys Asp Gln Ala Val Cys Gly Ser Cys Trp Ser Phe
           355                 360                 365
   Gly Thr Val Gly Glu Leu Glu Ala Ala Leu Phe Arg Lys Ala Arg Arg
       370                 375                 380
   Phe Ser Met Phe Thr Gly Lys Leu Thr Arg Leu Ser Glu Gln Gln Leu
   385                 390                 395                 400
   Val Asp Cys Ser Trp Asn Gln Gly Asn Asn Gly Cys Asp Gly Gly Glu
                   405                 410                 415
   Asp Phe Arg Ala Tyr Glu Tyr Ile Arg Ala His Gly Leu Ala Thr Asp
               420                 425                 430
   Glu Asp Tyr Gly Ala Tyr Leu Gly Gln Asp Gly Ile Cys His Asp Thr
           435                 440                 445
   Lys Val Asn Ala Thr Val Thr Thr Ile Lys Asn Tyr Ile Asn Val Thr
       450                 455                 460
   Asp Lys Glu Ser Leu Gln Lys Ala Leu Ala Asn Val Gly Pro Val Ser
   465                 470                 475                 480
   Val Ser Ile Asp Ala Ala Val Lys Ala Phe Thr Phe Tyr Ser Asn Gly
                   485                 490                 495
   Val Phe Tyr Asp Pro Lys Cys Arg Asn Asp Thr Asp Gly Leu Asp His
               500                 505                 510
   Ala Val Leu Ala Val Gly Tyr Gly Thr Leu Gln Gly Glu Pro Tyr Trp
           515                 520                 525
   Leu Ile Lys Asn Ser Trp Ser Thr Tyr Trp Gly Asn Asp Gly Tyr Val
       530                 535                 540
   Leu Ile Ser Gln Lys Asp Asn Asn Cys Gly Val Ala Ser Gln Gly Thr
   545                 550                 555                 560
   Tyr Val Glu Leu
<210> 7
   <211> 564
   <212> PRT
   <213> Rhipicephalus appendiculatus
<400> 7
   Met Met Arg His Ala Cys Ile Val Leu Leu Leu Ser Ile Ala Gly Cys
   1               5                   10                  15
   Leu Ala Gln Asp Leu Pro Asp Trp Gly Ser Phe Tyr Lys Val Lys Gly
               20                  25                  30
   Val Leu Tyr Leu Pro Tyr Ala Glu Ile Arg Glu Pro Phe Thr Gly Tyr
           35                  40                  45
   Tyr Asp Ala Thr Gln Asn Thr Ser Arg Ile Asp Tyr Tyr Asp Gly Met
       50                  55                  60
   Val Gln Thr Val Gln Leu Ser Pro Ser Ala Pro Ile Gly Gly Arg Arg
   65                  70                  75                  80
   Ile Pro Leu Trp His Gln Leu Gln Asp Cys Ile His Ala Arg Pro Lys
                   85                  90                  95
   Asp Met Gly Val Gln Ser Ala Pro Ala Ser Gln Val Asn Gly Thr Glu
               100                 105                 110
   Asn Ala His Leu Phe Pro Leu Gln Asp Val Leu Pro Lys Cys Cys Gly
           115                 120                 125
   Phe Thr Phe Val Arg Arg Glu Ser Cys Trp Phe Gly Asn Asp Asp Ala
       130                 135                 140
   Val Ala His Gln Gly Lys Arg His Cys Glu Arg Phe Gln Leu Thr Val
   145                 150                 155                 160
   Pro Thr Arg Asp Arg Val Ser Lys Tyr Thr Leu Trp Val Ser Arg Asp
                   165                 170                 175
   Ser Gln Gly Arg Ala Val Pro Arg Arg Tyr Leu Met Arg Gly Tyr Asn
               180                 185                 190
   Thr Leu Leu Gly Ser His Phe Asp Lys Tyr Glu Val Leu Tyr Tyr Gly
           195                 200                 205
   Tyr Ser Arg Asp Pro Val Pro Pro Ser Val Phe Asp Val Thr Thr Leu
       210                 215                 220
   Phe Asn Gly Thr Cys Arg Ser Phe Pro Gly Pro Gly Ala Glu Arg Leu
   225                 230                 235                 240
   Ala Leu His Asn Pro Met Ala Glu Phe Leu Gly Asn His Asp Gly His
                   245                 250                 255
   Thr Lys His Ser Phe Glu Asp Phe Lys Glu Thr His Lys Arg Thr Tyr
               260                 265                 270
   Glu Leu Asp Thr Glu His Asp Arg Arg Arg Asp Ile Phe Arg Gln Asn
           275                 280                 285
   Leu Arg Phe Ile Asp Ser Lys Asn Arg Ala Asn Leu Gly Tyr Asn Leu
       290                 295                 300
   Ala Val Asn His Leu Ala Asp Arg Thr Arg Glu Glu Ile Ser Val Leu
   305                 310                 315                 320
   Arg Gly Arg Leu Gln Ser Lys Asp Gly Ser Ser Arg Ala Glu Pro Phe
                   325                 330                 335
   Pro Arg His Arg Phe Thr Ala Lys Leu Pro Asp Gln Ile Asp Trp Arg
               340                 345                 350
   Pro Tyr Gly Ala Val Thr Pro Val Lys Asp Gln Ala Val Cys Gly Ser
           355                 360                 365
   Cys Trp Ser Phe Gly Thr Val Gly Glu Leu Glu Gly Ala Tyr Phe Arg
   370 375 380
   Lys Thr Gly Arg Leu Val Arg Leu Ser Glu Gln Gln Leu Val Asp Cys
   385 390 395 400
   Ser Trp Asn Asn Gly Asn Asn Gly Cys Asp Gly Gly Glu Asp Phe Arg
   405 410 415
   Ala Tyr Glu Tyr Ile Ala Asp His Gly Leu Ala Ser Asp Glu Asp Tyr
   420 425 430
   Gly Ala Tyr Ile Gly Gln Asp Gly Val Cys His Asp Ser Lys Val Asn
   435 440 445
   Ser Thr Ile Ser Ser Ile Lys Ser Tyr Val Asn Ile Thr Asn Arg Asp
   450 455 460
   Asp Leu Pro Thr Ala Leu Ala Asn Val Gly Pro Val Ser Val Ser Ile
   465 470 475 480
   Asp Ala Ala Leu Arg Ser Phe Ser Phe Tyr Pro Thr Val Ser Ser Met
   485 490 495
   Ile Pro Thr Ala Ala Met Asp Thr Asp Ser Leu Asp His Ser Val Leu
   500 505 510
   Arg Gln Ser Ala Thr Arg Thr Leu Gln Gly Glu Pro Tyr Trp Gly Val
   515 520 525
   Lys Asn Ser Trp Val Tyr Leu Leu Gly Glu Met Met Gly Tyr Val Leu
   530 535 540
   Ile Ser Pro Lys Gly Thr Thr Thr Gly Gly Val Ala Thr Gln Gly Thr
   545 550 555 560
   Tyr Val Glu Leu
<210> 8
   <211> 194
   <212> PRT
   <213> Metaseiulus occidentalis
<400> 8
   Met Asn Arg Leu Leu Tyr Ile Val Pro Leu Leu Phe Ala Val Val Ser
   1 5 10 15
   Ala Arg Leu Asp Asn Asp Val Pro Ser Glu Ser Ser Asn Lys Tyr His
               20                  25                  30
   Leu His Glu Thr Cys Arg Val Ala Leu Gln Asn Gln Ile Asp Arg Glu
           35                  40                  45
   Leu His Ala Ser Leu Val Tyr Gln Gln Met Ala Ala His Phe Glu Asn
       50                  55                  60
   Asn Lys Val Ala Arg Lys Gly Phe Ala Lys Phe Phe Met Asp Asn Ser
   65                  70                  75                  80
   Asn Glu Glu Arg Asp His Ala Gln Lys Leu Ile Ser Tyr Ile Asn Ser
                   85                  90                  95
   Arg Gly Gly Thr Ile Ala Ala Phe Arg Val Ser Met Pro Lys Asp Thr
               100                 105                 110
   Thr Trp Ala Ser Ala Arg Ala Ala Leu Glu Ser Ala Leu Glu Leu Glu
           115                 120                 125
   Ile Glu Val Asn Asn Ala Leu His Glu Val His Gly Lys Ala Glu Arg
       130                 135                 140
   Asp Cys Thr Asp Pro Gln Leu Gln Asp Phe Leu Glu Ala Asn Phe Leu
   145                 150                 155                 160
   Asn Glu Gln Val Glu Ser Ile Asp Asn Ile His Arg Leu Leu Ala Thr
                   165                 170                 175
   Leu Asn Gly Met Asp Gln Gly Leu Gly Glu Tyr Leu Val Asn Lys Asp
               180                 185                 190
   Leu Gln
<210> 9
   <211> 196
   <212> PRT
   <213> Ixodes ricinus
<400> 9
   Met Lys Gln Phe Val Val Ile Leu Ala Leu Ile Gly Ala Ala Thr Ser
   1               5                   10                  15
   Gly Asn Asn Leu Phe Glu Asn Leu Asp Lys Tyr Pro Leu Gln Asp Glu
               20                  25                  30
   Cys Gln Ala Ala Leu Gln Glu His Ile Asn Val Glu Met His Ala Ser
           35                  40                  45
   Leu Val Tyr Met Gln Met Ala Ala His Phe Asp Asn Asn Lys Val Ala
       50                  55                  60
   Arg Lys Gly Phe Ser Thr Phe Phe Ala Glu Asn Ser Lys Glu Glu Arg
   65                  70                  75                  80
   Glu His Ala Gln Lys Ile Ile Asp Tyr Ile Asn Lys Arg Gly Ser Thr
                   85                  90                  95
   Val Ser Leu Val Asn Ile Asp Met Pro Leu Ile Thr Thr Trp Lys Ser
               100                 105                 110
   Val Leu Gln Ala Leu Arg Asp Ala Ile Ser Leu Glu Asn Lys Val Thr
           115                 120                 125
   Asn Lys Leu His Ala Val His Lys Ile Ala Asp Glu Glu Cys Lys Asp
       130                 135                 140
   Pro Gln Leu Met Asp Phe Ile Glu Ser Glu Phe Leu Glu Glu Gln Val
   145                 150                 155                 160
   Asn Ser Ile Asp Lys Leu Gln Arg Met Ile Thr Val Leu Ser Asn Met
                   165                 170                 175
   Asp Ser Gly Thr Gly Glu Tyr Leu Leu Asp Arg Glu Leu Leu Gly Asp
               180                 185                 190
   Lys Lys Glu Phe
           195
<210> 10
   <211> 196
   <212> PRT
   <213> Ixides persulcatus
<400> 10
   Met Lys Gln Phe Val Val Leu Leu Ala Leu Ile Gly Ala Ala Thr Ser
   1               5                   10                  15
   Gly Asn Asn Leu Phe Glu Asn Leu Asp Lys Tyr Pro Leu Gln Asp Glu
               20                  25                  30
   Cys Gln Ala Ala Leu Gln Glu His Ile Asn Val Glu Met His Ala Ser
           35                  40                  45
   Leu Val Tyr Met Gln Met Ala Ala His Phe Asp Asn Asn Lys Val Ala
       50                  55                  60
   Arg Lys Gly Phe Ser Thr Phe Phe Ala Glu Asn Ser Lys Glu Glu Arg
   65                  70                  75                  80
   Glu His Ala Gln Lys Ile Ile Asp Tyr Ile Asn Lys Arg Gly Ser Thr
                   85                  90                  95
   Val Ser Leu Val Asn Ile Asp Met Pro Leu Ile Thr Thr Trp Lys Ser
               100                 105                 110
   Val Leu Gln Ala Leu Arg Asp Ala Ile Ser Leu Glu Asn Lys Val Thr
           115                 120                 125
   Asn Lys Leu His Ala Val His Lys Ile Ala Asp Glu Glu Cys Lys Asp
       130                 135                 140
   Pro Gln Leu Met Asp Phe Ile Glu Ser Glu Phe Leu Glu Glu Gln Val
   145                 150                 155                 160
   Asn Ser Ile Asp Lys Leu Gln Arg Met Ile Thr Val Leu Ser Asn Met
                   165                 170                 175
   Asp Ser Gly Thr Gly Glu Tyr Leu Leu Asp Arg Glu Leu Leu Gly Asp
               180                 185                 190
   Lys Lys Glu Phe
           195
<210> 11
   <211> 199
   <212> PRT
   <213> Heamaphysalis longicornis
<400> 11
   Met Leu Pro Ile Leu Ile Phe Ala Phe Ala Leu Leu Cys Val Ala Ser
   1               5                   10                  15
   Ala Gly Asn Asn Leu Asn Glu Gln Val Asn Gln Asn Lys Tyr Phe Leu
               20                  25                  30
   His Asp Arg Cys Arg Leu Gly Leu Gln Glu Gln Ile Asn Ala Glu Leu
           35                  40                  45
   His Ala Ser Leu Val Tyr Met Gln Met Ala Ala Tyr Leu Gly Asn Asn
       50                  55                  60
   Lys Val Ala Arg Ala Gly Phe Ala Arg Phe Phe Ser Asp Gln Ser Ser
   65                  70                  75                  80
   Glu Glu Arg Glu His Ala Gln Lys Leu Val Asp Tyr Val Asn Leu Arg
                   85                  90                  95
   Gly Gly Thr Val Ser Asn Val Asn Val Asp Met Pro Ala Thr Ala Thr
               100                 105                 110
   Trp Met Ser Val Leu Asp Thr Leu Gln Ala Ala Leu Ala Leu Glu His
           115                 120                 125
   Gln Val Thr Asn Arg Leu His Gly Leu His Ala Leu Ala Ala Asp His
       130                 135                 140
   Cys Arg Asp Pro Gln Met Thr Asp Phe Leu Glu Gln Glu Phe Leu Ala
   145                 150                 155                 160
   Glu Gln Met His Ser Ile Asp Lys Leu Gln Arg Leu Ile Thr Gln Leu
                   165                 170                 175
   Gln Asn Met Asp Thr Gly Leu Gly Glu Phe Leu Leu Asp Arg Glu Leu
               180                 185                 190
   Arg Arg Glu Asp Ser Asp Lys
           195
<210> 12
   <211> 21
   <212> DNA
   <213> Artificial Sequence
<220>
   <223> PCR Primer
<400> 12
   tgggcctctg ggatcagctc g 21
<210> 13
   <211> 21
   <212> DNA
   <213> Artificial Sequence
<220>
   <223> PCR Primer
<400> 13
   aatatcgcct cgttcggcgg c 21
<210> 14
   <211> 29
   <212> DNA
   <213> Artificial Sequence
<220>
   <221> misc_feature
   <222> (6)..(6)
   <223> d stands for A, G or T
<220>
   <221> misc_feature
   <222> (7)..(7)
   <223> w stands for A or T
<220>
   <221> misc_feature
   <222> (8)..(9)
   <223> s stands for C or G
<220>
   <221> misc_feature
   <222> (12)..(12)
   <223> s stands for C or G
<220>
   <221> misc_feature
   <222> (10)..(10)
   <223> y stands for C or T
<220>
   <221> misc_feature
   <222> (18)..(18)
   <223> y stands for C or T
<220>
   <221> misc_feature
   <222> (25)..(25)
   <223> h stands for A, C or T
<220>
   <221> misc_feature
   <222> (19)..(19)
   <223> m stands for A or C
<220>
   <221> misc_feature
   <222> (21)..(21)
   <223> r stands for A ro G
<220>
   <221> misc_feature
   <222> (24..(24)
   <223> r stands for A ro G
<220>
   <223> PCR Primer
<400> 14
   cacgcdwssy tsgthtaymw rcaratggc 29
<210> 15
   <211> 24
   <212> DNA
   <213> Artificial Sequence
<220>
   <221> misc_feature
   <222> (2)..(3)
   <223> r stands for A ro G
<220>
   <221> misc_feature
   <222> (6)..(6)
   <223> s stands for C or G
<220>
   <221> misc_feature
   <222> (8)..(8)
   <223> y stands for C or T
<220>
   <221> misc_feature
   <222> (9)..(9)
   <223> s stands for C or G
<220>
   <221> misc_feature
   <222> (10)..(11)
   <223> b stands for C, G or T
<220>
   <221> misc_feature
   <222> (15)..(15)
   <223> v stands for A, C or G
<220>
   <221> misc_feature
   <222> (17)..(17)
   <223> d stands for A, G or T
<220>
   <221> misc_feature
   <222> (21)..(21)
   <223> r stands for A or G
<220>
   <223> PCR Primer
<400> 15
   arraastysb bctcvadgaa rtcc 24
<210> 16
   <211> 21
   <212> DNA
   <213> Artificial Sequence
<220>
   <223> PCR Primer
<400> 16
   gtcaacacga acgcagcagc a 21
<210> 17
   <211> 21
   <212> DNA
   <213> Artificial Sequence
<220>
   <223> PCR Primer
<400> 17
   atctgcgcat ccagcgtcgt g 21
<210> 18
   <211> 21
   <212> DNA
   <213> Artificial Sequence
<220>
   <223> PCR Primer
<400> 18
   tattggtgct acaacaccgg c 21
<210> 19
   <211> 21
   <212> DNA
   <213> Artificial Sequence
<220>
   <223> PCR Primer
<400> 19
   gctggacagt attgtgacta t 21
<210> 20
   <211> 21
   <212> DNA
   <213> Artificial Sequence
<220>
   <223> PCR Primer
<400> 20
   atgttgatcc gctgcgttgt c 21
<210> 21
   <211> 21
   <212> DNA
   <213> Artificial Sequence
<220>
   <223> PCR Primer
<400> 21
   ctacagctcg acgtaggttg c 21
<210> 22
   <211> 21
   <212> DNA
   <213> Artificial Sequence
<220>
   <223> PCR Primer
<400> 22
   gtctggactt tatcgcctac c 21
<210> 23
   <211> 21
   <212> DNA
   <213> Artificial Sequence
<220>
   <223> PCR Primer
<400> 23
   ttcggtagcc tttcagcgtg c 21
<210> 24
   <211> 18
   <212> PRT
   <213> Dermanyssus gallinae
<400> 24
   Met Leu Ile Arg Cys Val Val Thr Ala Leu Ala Ala Val Thr Val Val
   1               5                   10                  15
   Ser Gly
<210> 25
   <211> 18
   <212> PRT
   <213> Dermanyssus gallinae
<400> 25
   Met Lys Arg Leu Val Cys Ile Leu Val Pro Leu Leu Phe Ala Ala Val
   1               5                   10                  15
   Leu Ala